# EUROPEAN PATENT SPECIFICATION

(11) **EP 2 852 841 B1**
(45) Date of publication and mention of the grant of the patent: **11.10.2017**
(21) Application number: 13794518.4
(22) Date of filing: 23.05.2013
(51) Int. Cl.: G01N 33/536, G01N 33/50, G01N 33/53, C12Q 1/68

(54) **MICROBEAD AGGLUTINATION BASED ASSAYS**
MIKROKÜGELCHENAGGLUTINIERUNGS-ASSAYS
DOSAGES SUR LA BASE DE L'AGGLUTINATION DE MICROBILLES

(30) Priority: 23.05.2012 US 201261650768 P; 02.06.2012 US 201261654861 P
(43) Date of publication of application: 01.04.2015
(73) Proprietor: King Abdullah University of Science and Technology, Thuwal 23955-6900 (SA)
(72) Inventor: FOULDS, Ian, G., Kelowna, BC V1Y 3Y2 (CA); KODZIUS, Rimantas, Thuwal 23955-6900 (SA); CASTRO SIGNORET, David, Agustin, Thuwal 23955-6900 (SA)
(74) Representative: Feray, Valérie
(86) International application number: PCT/IB2013/001846
(87) International publication number: WO 2013/175318

(56) References cited:
- WO-A2-2005/100602
- WO-A2-2010/144150
- WO-A2-2012/044387
- US-A- 5 817 526
- US-A- 5 989 813
- US-A1- 2010 119 132
- US-B1- 6 803 196

## Description

### CLAIM OF PRIORITY

This application claims the benefit of prior U.S. Provisional Patent Application No. 61/650,768, filed May 23, 2012, and U.S. Provisional Patent Application No. 61/654,861, filed June 2, 2012.

### TECHNICAL FIELD

This invention relates to an agglutination assay for detection of analytes in a sample. More specifically, this invention relates to a three-component agglutination assay and methods for determining the degree of aggregation resulting from an agglutination assay.

### BACKGROUND

For many years, due to the complexity of the testing, majority of laboratory testing was performed in a central laboratory. Recently, however, testing has emerged from the laboratory to the patient's bedside, the pharmacy, the physician's office, the patient's home and other non-laboratory sites. This testing is called point-of-care testing and is defined as testing at the point where patient care is given. Agglutination assays have been used for point-of-care testing. Agglutination assays rely upon agglutination of particles to which molecules (e.g. polypeptides or nucleic acids) are bound to indicate the presence of a corresponding analyte in the sample. In the presence of an analyte, the particles will aggregate or clump. Such clumps indicate a positive result, and in some circumstances, the clumps may be seen with the naked eye.

### SUMMARY

In one aspect, a method for detecting the presence of an analyte in a sample can include adding a plurality of microparticles of a first-type to the sample. Each microparticle of a first-type can include a first binding partner, which can be configured to interact with at least a first portion of an analyte.

In some embodiments, a method can include adding a plurality of microparticles of a second-type to the sample. Each microparticle of a second-type can include a second binding partner, which can be configured to interact with at least a second portion of the analyte. In some embodiments, a first portion of an analyte can be different from a second portion of an analyte.

Described herein, a microparticle of a first-type can be the same as a microparticle of a second-type. Further described herein, a microparticle of a first-type can be different than a microparticle of a second-type. The microparticles may be made of different materials, include different surface functionalization, or be a different size.

In some embodiments, a method can include identifying an aggregate including at least one microparticle of the first-type, at least one microparticle of the second-type and an analyte. An aggregate can indicate the presence of the analyte.

In some embodiments, a plurality of microparticles of a second-type can be added to a sample at the same time as a plurality of microparticles of a first-type is added to the sample. In other embodiments, a plurality of microparticles of the second-type can be added to a sample after a plurality of microparticles of a first-type is added to the sample. In some circumstances, a plurality of microparticles of the second-type can be added to a sample less than 10 minutes or less than 5 minutes after a plurality of microparticles of a first-type is added to the sample.

In other embodiments, a plurality of microparticles of the second-type can be added to a sample after a plurality of microparticles of a first-type is added to the sample, which can allow microparticles of a first-type bind to the analyte in the sample. As described herein, molecules of the analyte that are not bound to a microparticle of a first-type can be washed away from the sample or plurality of microparticles of a first-type. Following the wash step, a plurality of microparticles of a second-type can be added to the sample, allowing microparticles of the second-type to bind to a complex including at least one microparticle of a first-type and the analyte.

In some embodiments, a first portion of an analyte and a second portion of the analyte can have a common region. In other embodiments, a first portion of an analyte and a second portion of the analyte can be independent.

In some embodiments, an analyte can be a nucleic acid, a polypeptide or a polysaccharide. In some embodiments, a first binding partner or a second binding partner can be a nucleic acid or a polypeptide.

In some embodiments, a first binding partner can be associated with an at least one microparticle of a first-type through a covalent, ionic, van der Waals, dipolar or hydrogen bond. In some circumstances, a first binding partner can be associated with an at least one microparticle of a first-type through a covalent or ionic bond.

In some embodiments, a second binding partner can be associated with an at least one microparticle of a second-type through a covalent, ionic, van der Waals, dipolar or hydrogen bond. In some circumstances, a second binding partner can be associated with an at least one microparticle of a second-type.

In some embodiments, identifying an aggregate can include imaging a control in the absence of the analyte to create a control image. In some embodiments, identifying an aggregate can include assigning a color-scale value to each pixel of the control image based on the intensity of a color in the pixel. In some embodiments, identifying an aggregate can include calculating a distribution of the color-scale values assigned to the pixels in the control image.

In some embodiments, identifying an aggregate can include imaging the sample, which can include a plurality of microparticles of a first-type and a plurality of microparticles of a second-type to create a sample image. In some embodiments, identifying an aggregate can include assigning a color-scale value to each pixel of a control image based on an intensity of a color in the pixel. In some embodiments, identifying an aggregate can include calculating a distribution of color-scale values assigned to the pixels in a sample image.

In some embodiments, identifying an aggregate can include comparing a distribution of color-scale values assigned to the pixels in a sample image to a distribution of color-scale values assigned to the pixels in a control image. A wider distribution of the color-scale values assigned to the pixels in the sample image than the distribution of the color-scale values assigned to the pixels in the control image can indicate the presence of an aggregate.

In some embodiments, identifying an aggregate can include flowing at least one droplet of an agglutination mixture through a channel. In some embodiments, identifying an aggregate can include quantifying the agglutination by the distribution of the image's grayscale values. In some embodiments, the agglutination mixture can be formed by functionalized microbeads and the analyte. In some embodiments, the flow rate can be between 25 µL/min and 500 µL/min.

In some embodiments, the method can include measuring resistance of the agglutination mixture. As described herein, identifying an aggregate can include measuring the particles with a coulter counter.

In some embodiments, identifying an aggregate can include quantifying the degree of aggregation. In some embodiments, quantifying the degree of aggregation can include calculating the number of pixels assigned to each color-scale value.

In some embodiments, identifying an aggregate can include imaging the sample, which can include a plurality of microparticles of a first-type and a plurality of microparticles of a second-type to create a sample image.

In some embodiments, identifying an aggregate can include assigning a binary value to each pixel of a sample image based on the intensity of a color in the pixel.

In some embodiments, identifying an aggregate can include calculating the number of pixels in the sample image assigned to a first binary value. An assignment of the first binary value to any one pixel can indicate the presence of an aggregate.

In some embodiments, identifying an aggregate can include imaging a control in the absence of an analyte to create a control image.

In some embodiments, identifying an aggregate can include assigning a binary value to each pixel of a control image based on the intensity of a color in the pixel.

In some embodiments, identifying an aggregate can include calculating the number of pixels in a control image assigned to the first binary value.

In some embodiments, identifying an aggregate can include comparing the number of pixels in the sample image assigned to the first binary value with the number of pixels in the control image assigned to the first binary value. The presence of an aggregate can be indicated when the number of pixels in the sample image assigned to the first binary value is greater than the number of pixels in the control image assigned to the first binary value.

In some embodiments, identifying an aggregate includes quantifying the degree of aggregation. In some embodiments, identifying an aggregate can include quantifying the degree of aggregation includes calculating the percentage of pixels in the image of the sample assigned to the first binary value.

As described herein, a method for determining the presence of an aggregate can include imaging a control in the absence of an analyte to create a control image. In some embodiments, a method for determining the presence of an aggregate can include assigning a color-scale value to each pixel of the control image based on the intensity of a color in the pixel. As described herein, a method for determining the presence of an aggregate can include calculating a distribution of the color-scale values assigned to the pixels in the control image.

As described herein, a method for determining the presence of an aggregate can include imaging a sample. A sample can include a plurality of a first-type of microparticles and a plurality of a second-type to create a sample image. Each microparticle of the first-type can include a first binding partner configured to interact with at least a first portion of the analyte. Each microparticle of the second-type can include a second binding partner configured to interact with at least a second portion of the analyte. The first portion of the analyte can be different from the second portion of the analyte. As described herein, a method for determining the presence of an aggregate can include assigning a color-scale value to each pixel of the sample image based on the intensity of a color in the pixel. As described herein, a method for determining the presence of an aggregate can include calculating a distribution of the color-scale values assigned to the pixels in the sample image.

As described herein, a method for determining the presence of an aggregate can include comparing the distribution of the color-scale values assigned to the pixels in the sample image to the distribution of the color-scale values assigned to the pixels in the control image, wherein a wider distribution of the color-scale values assigned to the pixels in the sample image than the distribution of the color-scale values assigned to the pixels in the control image indicates the presence of an aggregate.

As described herein, a method for determining the presence of an aggregate can include quantifying the degree of aggregation. Quantifying the degree of aggregation can include calculating the number of pixels assigned to each color-scale value.

As described herein, a method for determining the presence of aggregates can include imaging a sample, which can including a plurality of microparticles of a first-type and a plurality of microparticles of a second-type to create a sample image. Each microparticle of a first-type can include a first binding partner configured to interact with at least a first portion of an analyte. Each microparticle of a second-type can include a second binding partner configured to interact with at least a second portion of the analyte. A first portion of the analyte can be different from a second portion of the analyte.

As described herein, a method for determining the presence of aggregates can include assigning a binary value to each pixel of the sample image based on the intensity of a color in the pixel.

As described herein, a method for determining the presence of aggregates can include calculating the number of pixels in the sample image assigned to a first binary value. An assignment of the first binary value to a pixel can indicates the presence of an aggregate.

As described herein, a method for determining the presence of aggregates can include imaging a control in the absence of the analyte to create a control image. As described herein, a method for determining the presence of aggregates can include assigning a binary value to each pixel of a control image based on the intensity of a color in the pixel. As described herein, a method for determining the presence of aggregates can include calculating the number of pixels in a control image assigned to the first binary value.

As described herein, a method for determining the presence of aggregates can include comparing the number of pixels in a sample image assigned to the first binary value with the number of pixels in a control image assigned to the first binary value. The presence of an aggregate can be indicated when the number of pixels in the sample image assigned to the first binary value is greater than the number of pixels in the control image assigned to the first binary value.

As described herein, a method for determining the presence of aggregates can include quantifying the degree of aggregation. As described herein, quantifying the degree of aggregation can include calculating the percentage of pixels in the image of the sample assigned to the first binary value.

In some embodiments of each of the aspects above, a method a first binding partner, a second binding partner and an analyte can be nucleic acids. As described herein, identifying an aggregate or determining the presence of an aggregate can include annealing a first binding partner with a first portion of the analyte. As described herein, identifying an aggregate or determining the presence of aggregates can include annealing a second binding partner with a second portion of the analyte. As described herein, identifying an aggregate or determining the presence of aggregates can include ligating a first binding partner with a second binding partner to form a nucleic acid with a microparticle of a first-type and a microparticle of a second-type. As described herein, identifying an aggregate or determining the presence of aggregates can include denaturing the nucleic acid formed by the ligation of the first binding partner with the second binding partner from the analyte.

In another aspect, a composition can include an analyte, at least one microparticle of a first-type, and at least one microparticle of a second-type. Each microparticle of a first-type can include a first binding partner configured to interact with at least a first portion of an analyte. Each microparticle of a second-type can include a second binding partner configured to interact with at least a second portion of an analyte.

In some embodiments, a first portion of an analyte can be different from a second portion of the analyte. In some embodiments, a first portion of an analyte and a second portion of the analyte can have a common region. In some embodiments, a first portion of the analyte and a second portion of an analyte can be independent.

In some embodiments, an analyte can be a nucleic acid, a polypeptide or a polysaccharide. In some embodiments, a first binding partner or a second binding partner can be a nucleic acid or a polypeptide.

In some embodiments, a first binding partner can be bound to at least one microparticle of a first-type. In some embodiments, a first binding partner can be covalently bound to at least one microparticle of a first-type.

In some embodiments, a second binding partner can be bound to at least one microparticle of a second-type. In some embodiments, a second binding partner can be covalently bound to at least one microparticle of a second-type.

As described herein, a first binding partner can be ligated to a second binding partner to form a nucleic acid, which can include a microparticle of a first-type and a microparticle of a second-type.

Other features or advantages will be apparent from the following detailed description of several embodiments, and also from the appended claims.

### BRIEF DESCRIPTION OF THE DRAWINGS

FIGS. 1a and 1b are schematics of an agglutination assay.
FIGS. 2a and 2b are schematics of a stepped-agglutination assay.
FIG. 3 is a schematic of a cycling ligation based agglutination assay.
FIG. 4 is a schematic of a ligation based agglutination assay.
FIG. 5a is a schematic of particles.
FIG. 5b is an image of a sample without agglutination.
FIG. 5c is an image of a sample with agglutination.
FIG. 5d is a schematic of particles in an aggregate with an analyte.
FIG. 6a is an image of a sample without agglutination.
FIG. 6b is a histogram of the pixel color-intensity from an image of a sample without agglutination.
FIG. 6c is an image of a sample with agglutination.
FIG. 6d is a histogram of the pixel color-intensity from an image of a sample with agglutination.
FIG. 7 is a binary image of a sample with agglutination.
FIG. 8a is a sequence of sample images showing agglutination with varying concentrations of analyte.
FIG. 8b is a schematic of a two-component agglutination assay.
FIG. 9a is a sequence of sample images showing agglutination with varying concentrations of analyte.
FIG. 9b is a schematic of a three-component agglutination assay.
FIGS. 10a and 10b are schematics of self-terminating agglutination assays.
FIGS. 11a and 11b are schematics of saturation during an agglutination assays.
FIG. 12 is a schematic of a non-agglutinate.
FIG. 13a is a sequence of sample images showing agglutination with varying concentrations of analyte.
FIG. 13b is a schematic of an agglutination assay.
FIG. 14 is a graph comparing different agglutination assays.
FIGS. 15a-e are pictures of a microfluidic device.
FIG. 16 is a schematic representation of the experimental setup for quantification of droplet-based agglutination assays.
FIG. 17 shows the representation of the quantification of agglutination.
FIGS. 18(a)-(c) show agglutination strength relating to target concentration; FIG. 18(d) shows the dependence of agglutination peak, assay detection limit and observable agglutination on microbeads concentration; FIG. 18(e) shows images at different concentrations.

### DETAILED DESCRIPTION

Agglutination tests can be based on aggregation of particles, for example, microparticles, in the presence a specific analyte. This aggregation can enable macroscopic observation. Agglutination-based tests can be used to explore the antibody-antigen reactions. *See* Bains W, Noble P: Sensitivity limits of latex agglutination tests. Am Clin Lab 12(3), 14, 16-17 (1993). Agglutination can also be used for protein assays utilizing protein tags or labels (e.g. a biotin/streptavidin two-component assay). *See* Moser Y, Lehnert T, Gijs MA: On-chip immuno-agglutination assay with analyte capture by dynamic manipulation of superparamagnetic beads. Lab on a chip 9(22), 3261-3267 (2009); D. C. Leslie, J. Li, B. C. Strachan, M. R. Begley, D. Finkler, L. A. Bazydlo, et al., "New detection modality for label-free quantification of DNA in biological samples via superparamagnetic bead aggregation," J Am Chem Soc, vol. 134, pp. 5689-96, Mar 28 2012; C. K. Tison and V. T. Milam, "Reversing DNA-Mediated adhesion at a fixed temperature," Langmuir, vol. 23, pp. 9728-9736, Sep 11 2007. Agglutination can also be used with assays utilizing natural binding partners. Additionally, agglutination can be used with assays utilizing nucleic acid-protein binding or nucleic acid hybridization. For example, agglutination assays have typically been two-component assays. However, two-component assays can be prone to self-termination of the linking analyte, and thus, can have a lower sensitivity. *See* Moser Y, *et al.* (2009); Vollenhofer-Schrumpf S, Buresch R, Schinkinger M: A simple nucleic acid hybridization/latex agglutination assay for the rapid detection of polymerase chain reaction amplicons, Journal of microbiological methods 68(3), 568-576 (2007). Beads can be used in agglutination assays. On-chip immune-agglutination assay by dynamic magnetic actuation was performed using 1 µm diameter superparamagnetic beads. *See* Moser, Y., *et al.* (2009). Three-component assays have been used with DNA hybridization; however, the current three-component assays can require up to 48 hours for incubation. See Rogers PH, Michel E, Bauer CA et al.: Selective, controllable, and reversible aggregation of polystyrene latex microspheres via DNA hybridization. Langmuir : the ACS journal of surfaces and colloids 21(12), 5562-5569 (2005).

While analyte detection is possible using the conventional agglutination assays, these assays can take a long time (up to 48 hours or more), can require significant sample volumes to obtain reliable results, be prone to self-termination and lack the requisite sensitivity. Microfluidics can decrease the detection volume required, can increase the

sensitivity and can provide a faster response for quantitative assay. For example, a simple agglutination assay on white latex cards was performed by Austrian research company, SY-LAB Garaete GMBH. *See* Vollenhofer-Schrumpf S, *et al.* (2007). DNA-based agglutination appeared to be improved by performing the assay in microfluidics. *See* Degre G, Brunet E, Dodge A, Tabeling P: Improving agglutination tests by working in microfluidic channels. Lab Chip 5(6), 691-694 (2005). In addition to DNA-based agglutination, ABO, D blood typing and subtyping has been performed using plug-based microfluidics. Antibody-based detection in microfluidics has also been performed and can require only microliter volumes of blood. The same plug-based platform has been used to detect bacteria and virus. *See* Kline TR, Runyon MK, Pothiawala M, Ismagilov RF: ABO, D blood typing and subtyping using plug-based microfluidics. Anal Chem 80(16), 6190-6197 (2008).

However, a need still exists for improved agglutination assays. The methods described herein can be performed in 5 minutes, can have increased sensitivity (for example, an order of magnitude improvement in sensitivity) over two-component assays, can have an increased measurement range, can eliminate or significantly decrease the saturation effects (i.e. self-termination effects) and can be used at room temperature. All of these features can make the described methods real candidates for point-of-care (POC) testing. Additionally, a strength of these methods can be that any molecule that can be bonded to at two or more points can be detected in this fashion. Moreover, methods for quantifying the amount of agglutination, which is dependent on the amount of analyte present in the sample, are described. These methods can also improve the speed with which results can be obtained and the sensitivity of the assays.

In sum, these methods can be used to detect the presence of atoms and molecules, such as proteins, carbohydrates or specific DNA sequences, at room temperature in just a few minutes. The described methods can be based on aggregation of particles, more specifically microparticles, in the presence of a specific analyte, which can enable the macroscopic observation.

As used herein, the terms agglutination, aggregation and clumping should be considered interchangeable.

In general, a method for detecting the presence of an analyte in a sample (i.e. an agglutination assay) can include two or more components. Components can be particles and/or analyte that can interact to cause agglutination. A component can also be a type of particle. A two component system can use analyte and multiple beads of the same type. A three component system can include 1 analyte and two different types of beads. In a two-component assay, for instance, the two components can be a particle and an analyte. Preferably, a method can include three or more components. In a three-component assay, the three components can be two particles and an analyte. Three-component assays can be performed by adding the particles simultaneously or in a step-wise fashion.

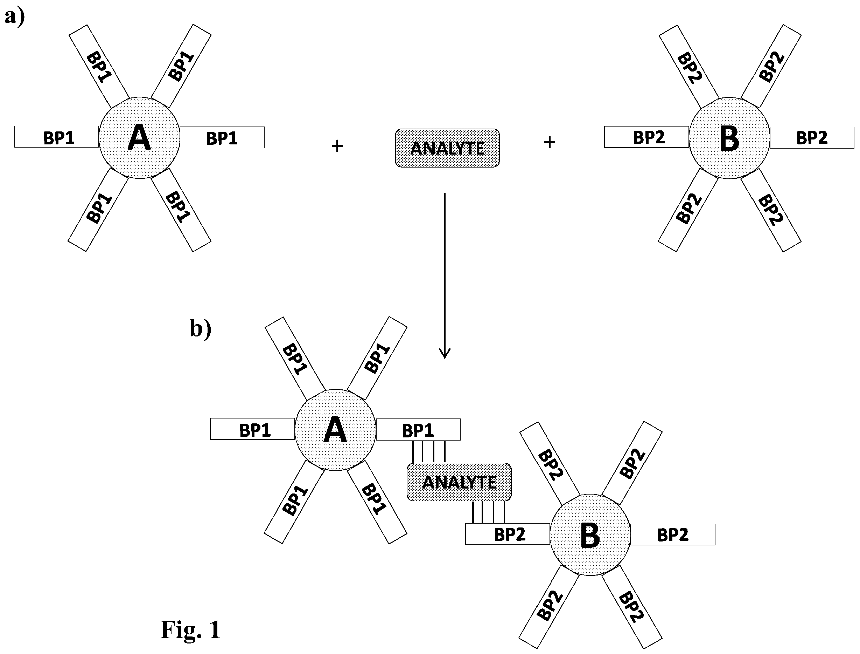

In particular, aggregates can be formed when particles are added to a sample including a target analyte. For example, when a particle of a first-type including a first binding partner and a particle of second-type including a second binding partner are added to a sample simultaneously (Fig. 1a), the analyte can bind with the first binding partner and the second binding partner (Fig. 1b). Aggregates of the target analyte and particles of the first-type and/or the second-type can form as this process is amplified, with additional molecules of the binding partners interacting with additional molecules of the analyte.

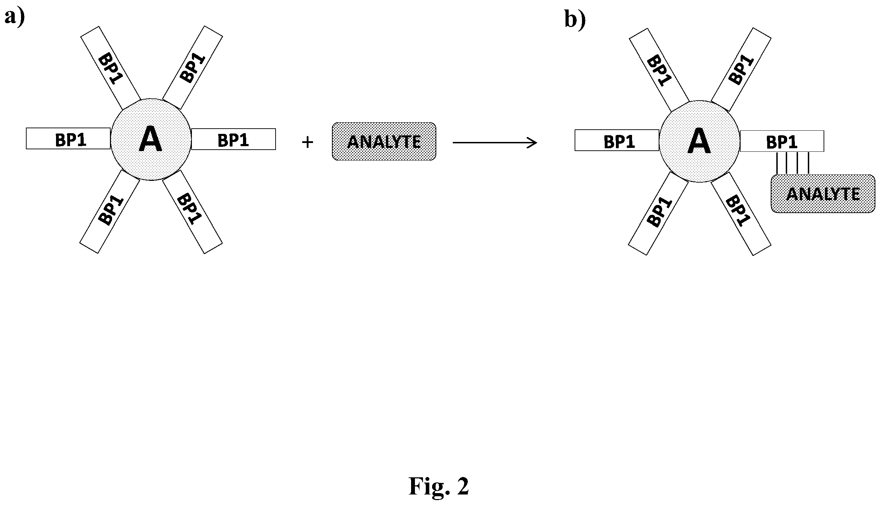

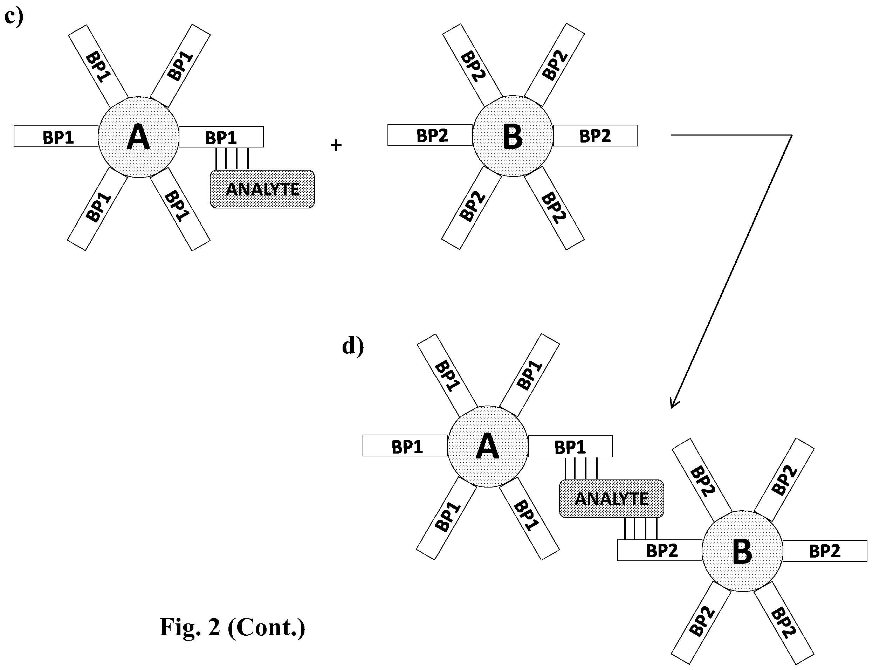

Alternatively, a particle of a first-type can include a first binding partner (Fig. 2a). As discussed above, including a first binding partner can mean that the particle of a first-type includes more than one molecule of the first binding partner. In the presence of the target analyte, the first binding partner can bind to at least one copy of the target analyte (Fig. 2b). Aggregates of the target analyte and particles of the first-type can form as this process is amplified, with additional molecules of the first binding partners interacting with additional molecules of the analyte (not shown). However, the addition of particles of a first-type to the sample may not result in aggregates. The presence of aggregates at this stage can depend on the ratio of particles of a first-type to analyte. The ratio can be based on number of molecules, concentration, etc. Unbound molecules of the analyte can be washed away following the first step (not shown). Unbound analyte may be present if the concentration of analyte or total number of analyte molecules is high. A particle of a second-type including a second binding partner can then be added to a sample (Fig.2c). Because the particles of the second-type have not been saturated with analyte, these particles can agglomerate with the saturated particles of a first-type or with free analyte (Fig. 2d). This can allow the maintenance of a high agglutination signal even at very high analyte concentrations.

Even more specifically, a method for detecting the presence of an analyte in a sample can include adding a plurality of particles of a first-type to a sample. A method can also include adding a plurality of particles of a second-type to the sample. A plurality of particles can include two or more particles.

With any of the described methods, samples can be *in vivo, ex vivo* or *in vitro* samples. Samples can include body fluids, for example, stool, saliva, sputum, bronchial lavage, drainage fluids (feces, urine), vaginal and nasal swabs, biopsy tissues, tears, breath, blood, serum (or better plasma), cerebrospinal, peritoneal, pleural, pericardial and joint fluids, maternal plasma (cell-free fetal nucleic acids) or amniotic fluid (fetal DNA or RNA). A sample can include tissues or cells. Samples can also include cellular components, such as nucleic acids, polypeptides, polysaccharides or small molecules. More specifically, nucleic acids can include DNA (e.g. gDNA, ssDNA, dsDNA, or zDNA) or RNA (e.g. cellular RNA, mRNA, or tRNA). Samples can also include pathogens (e.g. viruses or bacteria) or pathogen components, such as viral nucleic acids (e.g. viral DNA or viral RNA) or polypeptides (e.g. capsid proteins, envelope proteins or enzymes). *In vitro* samples can include food, water or *in vitro* experiments mimicking *in vivo* systems, for example, PCR reactions.

Particles can be made of any acceptable material, for example, ceramics, plastics, glass or metals. More specifically, particles can be made of latex, silica, melamine resin, polystyrene, polymethacrylate, polyamide, polyvinyl chloride, polyethylene, polyester, polypropylene, polycarbonate, polyacrylamide or polyvinyl alcohol. In preferred embodiments, the particles themselves are inert so that they do not - in the absence of a modification, e.g., a binding partner (see below) - interact with components of a sample. Preferentially, particles used with biological samples can be biocompatible which can mean that the particle can be well tolerated by an organism, tissue or cell. More specifically, biocompatibility can mean that a particle does not elicit an immune response when it is brought in contact with an organism, tissue or cell. It can also mean that a particle can integrate into cell structures, cells, tissues or organs of an organism. The organism can be mammal, in particular, a human.

Particles can also include a core with a coating. For example, a particle can include a metal core coated with a ceramic or plastic. Particles may also have additional features, such as being magnetic or luminescent. Particles may include a dye.

A particle of a first-type can be the same as a particle of a second-type, or a particle of a first-type can be different than a particle of a second-type The particles may be different for any number of reasons, including but not limited to, the particles being made of different materials, the particles including different surface functionalization, or the particles being a different size.

Particles can be microparticles, for example, can be microspheres or microbeads. Microparticles can include particles that are at least 0.01 µm, at least 0.1 µm, at least 0.25 µm, at least 0.5 µm, at least 1 µm, at least 5 µm, at least 10 µm, at least 25 µm, or at least 50 µm. Microparticles can include particles that are at most 500 µm, at most 250 µm, at most 100 µm, at most 75 µm, at most 50 µm, at most 25 µm, at most 10 µm, or at most 5 µm. Preferentially, microparticles are between 0.1 µm and 100 µm.

Each particle of a first-type can include a first binding partner, which can be configured to interact with at least a first portion of an analyte. For example, a first binding partner can be bound to a particle of the first-type. The bond between the first binding partner and the particle of the first-type can be a covalent, ionic, van der Waals, dipolar or hydrogen bond. Moreover, each particle of a second-type can include a second binding partner, which can be configured to interact with at least a second portion of an analyte. A second binding partner can be bound to a particle of the second-type. The bond between the second binding partner and the particle of the second-type can be a covalent, ionic, van der Waals, dipolar or hydrogen bond. To assist with binding of a binding partner to a particle, a particle can have a functionalized surface. A surface can be functionalized with, for example, NH₂, COOH, alkyl-OH, NR³⁺, acrylate, CHO, epoxy, SO₃H, SiO₂, NTA, EDTA, DTPA, NHS, TiO₂, TMS, avidin, streptavidin, protein A, albumin (BSA), collagen, chitosan, Pd-, Ag-, Au-, Pt-, SH, Ni-NTA, Al₂O₃, PEG300, PEG-NH₂, PEG-COOH, PEI, glutathione, SH, maleimide, an unbranched C₂-C₂₀ hydrocarbon chain or a branched C₂-C₃₀ hydrocarbon chain..

A binding partner can be configured to interact with at least a portion of an analyte. An analyte can be virtually any atom, ion or molecule, as long as it is known to have a binding partner that recognizes the analyte. An analyte can include nucleic acids, polypeptides, polysaccharides or small molecules. A binding partner (either the first or second) can also include a nucleic acid, polypeptide, polysaccharide or small molecule.

A nucleic acid can include at least 10 base pairs, at least 20 base pairs, at least 50 base pairs, at least 100 base pairs, at least 250 base pairs, at least 500 base pairs or at least 1000 base pairs. A nucleic acid can include at most 5000 base pairs, at most 3000 base pairs, at most 2000 base pairs, at most 1000 base pairs, at most 500 base pairs, at most 250 base pairs, at most 100 base pairs, at most 50 base pairs. A nucleic acid can include DNA (e.g. viral DNA, gDNA, ssDNA, dsDNA, or zDNA) or RNA (e.g. viral RNA, cellular RNA, mRNA, or tRNA).

A polypeptide can be a peptide, a polypeptide or a protein. For example, a polypeptide can include at least 2, at least 5, at least 10, at least 25, at least 50, at least 100, at least 250, at least 500 or at least 750 amino acids. A polypeptide can include at most 1000, at most 750, at most 500, at most 250, at most 100, at most 50, at most 25 or at most 15 amino acids. A polypeptide can include a full length protein, a fragment of a protein or a protein domain. A polypeptide can be a fusion protein, which can include portions originating from one protein or portions originating from more than one protein. A polypeptide can include a protein tag or marker. Protein tags and markers are well-known in the art. *See*, e.g., Lichty, et al., Comparison of affinity tags for protein purification, Protein Expr Purif., 41(1):98-105, May 2005. A polypeptide can also be modified, for example, by glycosylation, acetylation, alkylation, methylation, glutamylation, glycylation, isoprenylation, lipoylation, phosphopantetheinylation, phosphorylation, sulfation, selenation, ubiquitination, PEGylation, SUMOylation or biotinylation. A polypeptide can be a cellular polypeptide (e.g. prokaryotic or eukaryotic) or a viral polypeptide (e.g. capsid proteins, envelope proteins or enzymes). A polypeptide can include at least a portion of an antibody. More specifically, a polypeptide can include the Fᵥ region or the F_{ab} region.

A polysaccharide can include a starch, a glycogen, a cellulose, a pectin, a chitin or a glucan (e.g. β-glucan). A polysaccharide can be associated with a polypeptide.

A small molecule can include organic chemicals (e.g. pesticides or herbicides or vitamins). Examples of vitamins can include vitamin C, vitamin A, vitamin D, vitamin K, vitamin B₁, vitamin B₂, vitamin B₃, vitamin B₅, vitamin B6, vitamin B₇ (also known as biotin), vitamin B₉, vitamin B₁₂ or any derivative or analog thereof.

A binding partner can be an aptamer. A particle of a first-type and a particle of a second-type can be different but be used for recognition of same target. The aptamers can have robust binding to virtually any target.

The interaction can be binding, for example, selective binding. Selective binding can provide the selectivity needed to bind to the corresponding binding partner (or relatively small group of related molecules or proteins) in a complex mixture. The degree of binding can be less than 100%, less than 90%, less than 80%, less than 70%, less than 60%, less than 50%, less than 40%, less than 30%, less than 20% or less than 10% of a second binding partner present binding to a first binding partner. The degree of binding can be more than 10%, more than 20%, more than 30%, more than 40%, more than 50%, more than 60%, more than 70%, more than 80% or more than 90% of a second binding partner present binding to a first binding partner. A first binding partner and a second binding partner can bind with a dissociation constant less than 1 mM, less than 0.1 mM, less than 0.01 mM, less than 1 µM, less than 0.1 µM, or less than 0.01 µM. A first binding partner and a second binding partner can bind with a dissociation constant greater than 1nM, greater than 0.01 µM, greater than 0.1 µM, greater than 1 µM, greater than 0.01 mM, or greater than 0.1 mM.

The interaction can also be specific binding. Specific binding can be used to distinguish a binding partner from most other chemical species except optical isomers, isotopic variants and perhaps certain structural isomers. The degree of binding can be less than 100%, less than 90%, less than 80%, less than 70%, less than 60%, less than 50%, less than 40%, less than 30%, less than 20% or less than 10% of an analyte present binding to a capture protein. The degree of binding can be more than 10%, more than 20%, more than 30%, more than 40%, more than 50%, more than 60%, more than 70%, more than 80% or more than 90% of an analyte present binding to a capture protein. An analyte and a capture protein can bind with a dissociation constant less than 1 mM, less than 0.1 mM, less than 0.01 mM, less than 1 µM, less than 0.1 µM, or less than 0.01 µM. An analyte and a capture protein can bind with a dissociation constant greater than 1nM, greater than 0.01 µM, greater than 0.1 µM, greater than 1 µM, greater than 0.01 mM, or greater than 0.1 mM.

A first binding partner can be configured to interact with at least a first portion of an analyte. A second binding partner can be configured to interact with at least a second portion of an analyte. A first portion of an analyte can be different than a second portion of an analyte. A first portion of an analyte may include a common region with a second portion of an analyte. In other words, a first portion of an analyte may overlap with a second portion of an analyte. However, if that is the case, self-termination of an agglutination assay may arise. Therefore, preferentially, a first portion of an analyte and a second portion of an analyte are independent. In other words, a first portion of an analyte and a second portion of analyte do not overlap. This can prevent one microparticle from reducing or eliminating access to the analyte by another microparticle. Said differently, having a first portion of an analyte and a second portion of analyte that do not overlap can minimize actual or steric hindrance, shielding or repulsion.

A portion of an analyte can be a sequence (e.g. a nucleic acid sequence or an amino acid sequence), a secondary structure, a tertiary structure, a region or a domain. If the analyte is a complex (e.g. nucleic acid-nucleic acid, nucleic acid-polypeptide, polypeptide-polypeptide or polypeptide-polysaccharide), a portion of an analyte can be a sequence, a secondary structure, a tertiary structure, a region or a domain on one component of the complex or a sequence, a secondary structure, a tertiary structure, a region or a domain formed by more than one component of the complex.

In some circumstances, the plurality of particles of the second-type can be added to the sample at the same time as the plurality of particles of the first-type is added to the sample. In other words, the plurality of particles of the first-type and the plurality of particles of the second-type can be added simultaneously to the sample. However, simultaneous addition of the plurality of particles of the first-type and the plurality of particles of the second-type can result in active site saturation and/or loss of agglutination at high analyte concentrations. To resolve these issues, the plurality of particles of the second-type can be added to the sample after the plurality of particles of the first-type is added to the sample. For example, the plurality of particles of the second-type can be added to the sample less than 12 hours, less than 6 hours, less than 2 hours, less than 1 hour, less than 30 minutes, less than 15 minutes, less than 10 minutes, less than 5 minutes, less than 4 minutes, less than 3 minutes, less than 2 minutes or less than 1 minute after the plurality of particles of the first-type is added to the sample.

At the time the particles are added to the sample, the temperature of the sample may not need to be regulated, as the assay can rapidly occur at ambient temperatures (e.g. room temperature). The temperature of the sample, with or without the particles, can be at least 0°C, at least 10°C, at least 20°C, at least 25°C, at least 30°C, at least 35°C, at least 40°C, at least 50°C, at least 65°C or at least 75°C. The temperature of the sample, with or without the particles, can be at most 150°C, at most 125°C, at most 100°C, at most 75°C, at most 50°C, at most 40°C, at most 35°C, at most 30°C, at most 25°C, at most 20°C or at most 15°C. Room temperature can be considered at least 15°C and at most 30°C, at least 20°C and at most 25°C, or at least 20°C and at most 30°C.

A method can include identifying an aggregate including at least one microparticle of the first-type, at least one microparticle of the second-type and the analyte. Identifying an aggregate includes determining the presence of an aggregate or aggregates, and determining the degree of aggregation (e.g. the concentration of aggregates in the total sample or the quantity of aggregates in the sample). The identification of an aggregate can indicate the presence of the analyte. Because an aggregate or clump should only form in the presence of at least one particle and the analyte, if there is no analyte in the sample, there should be no aggregates. Conversely, if analyte is present in the sample, aggregates including at least one particle and the analyte should form. Aggregation can be promoted by including more than one binding partner on a particle. In fact, a particle may include at least 10, at least 100, at least 1000, at least 10,000, at least 100,000 or at least 1,000,000 molecules of its corresponding binding partner.

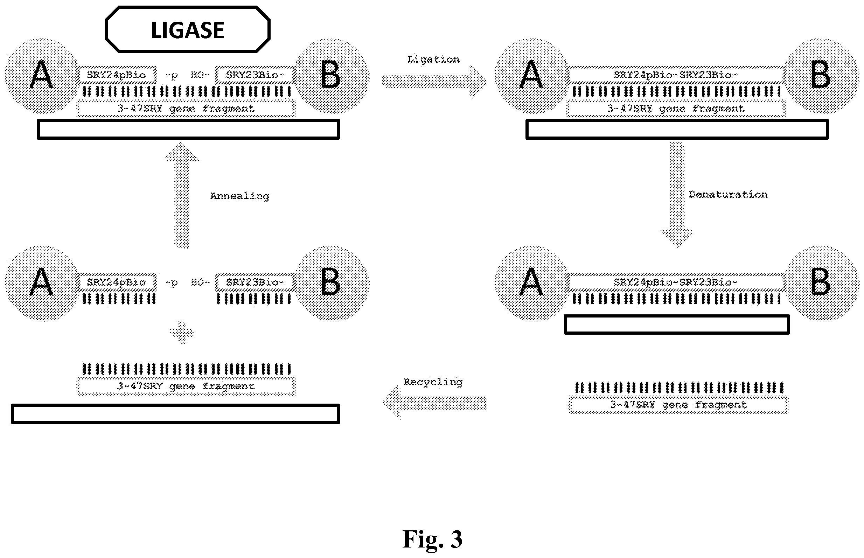

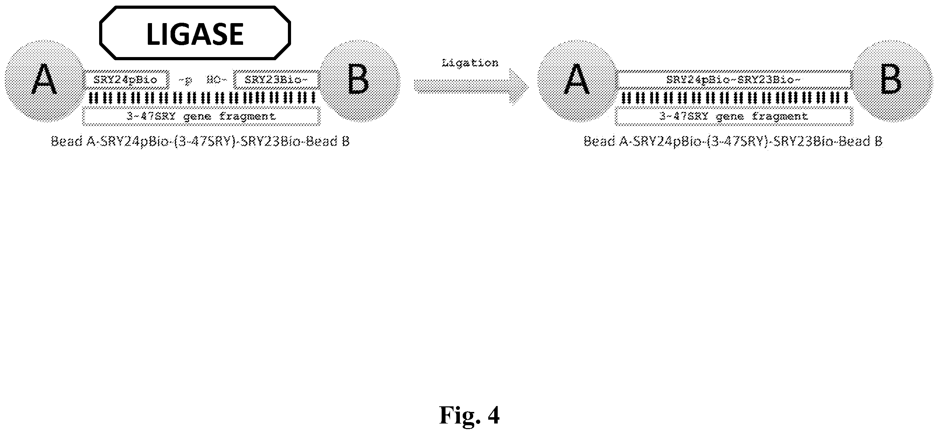

In some embodiments, the first binding partner, the second binding partner and the analyte can be nucleic acids. In that case, identifying an aggregate can include annealing a first binding partner with a first portion of the analyte and/or annealing the second binding partner with the second portion of the analyte (Fig. 3, Annealing). If the first binding partner and the second binding partner are annealed to the complementary target analyte and in close proximity to each other, the first binding partner can be ligated with the second binding partner to form a nucleic acid with a microparticle of the first-type and a microparticle of the second-type (Fig. 3, Ligation; *see also* Fig. 4). This process can have the benefits of stabilizing aggregates for detection. To amplifying the analyte detection signal and increase the sensitivity of the method, the analyte can be recycled. To recycle the analyte, the temperature of the sample can be raised to denature the double stranded nucleic acid formed by the analyte annealing with the binding partners (Fig 3, Denaturation). The analyte is then free to anneal with another set of binding partners (Fig. 3, Recycling).

As the methods described herein occur more rapidly than the methods previously known in the art, the time between addition of the plurality of particles of the first-type (i.e. the first particles added to the sample) and the time when aggregates can be identified can be less than 12 hours, less than 6 hours, less than 2 hours, less than 1 hour, less than 30 minutes, less than 15 minutes, less than 10 minutes, less than 5 minutes, less than 4 minutes, less than 3 minutes, less than 2 minutes less than 1 minute, less than 30 seconds or less than 15 seconds.

In general, identifying an aggregate can include imaging a sample. Imaging a sample can include capturing a still-image of the sample or capturing a video of the sample. In some cases, a video of the sample can be captured and a still-image can be extracted from the video images. A video can be a high-definition video. An image of a sample can include pixels. A pixel can be any of the small, discrete elements that constitute a visual representation of the sample.

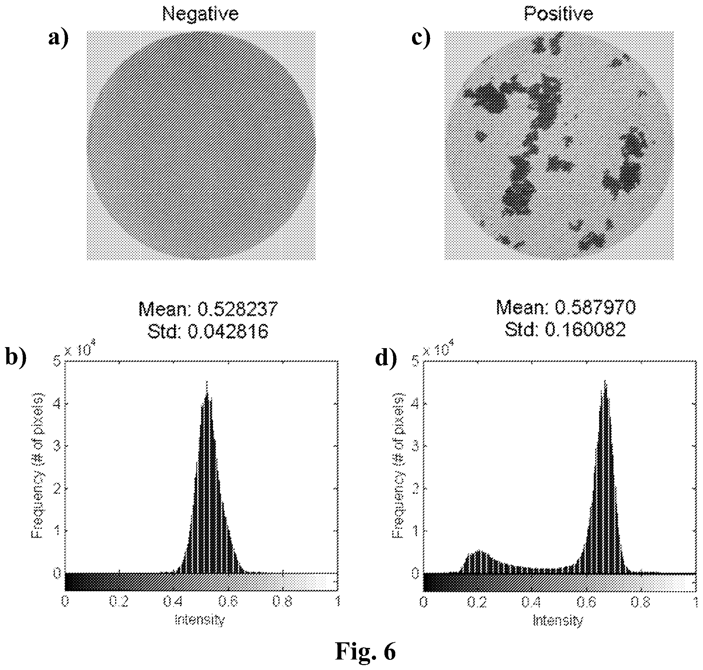

Under a first approach, identifying an aggregate can be a based on a distribution of the color-values of the pixels of the sample image. A color-value can be assigned to each pixel based on the intensity of the color within the pixel. For example, the color-values can be measured using grayscale values (ranging from 0 for black to 1 for white). If an analyte is not present in a sample (i.e. a negative sample), the image can have a narrow distribution of color-values, which tend to occur around the middle of the grayscale (e.g. the mean of the distribution) (Fig. 6a and 6b). In other words, the pixel color-values can have a low standard deviation. If an analyte is present in a sample (i.e. a positive sample), the image can have a wide distribution of color values (Fig. 6c and 6d). Said another way, the pixel color-values can have a higher standard deviation.

Additionally, the degree of aggregation can also be measured using this approach. For example, a decrease in the percentage of pixels at or near the mean of the distribution can indicate that the degree of agglutination is increased (Figs. 6a, 6b, 6c and 6d). An increase in the percentage of pixels at or near the extremes of the distribution can also indicate that the degree of agglutination is increased. An increase in the degree of aggregation can indicate the presence of the analyte and/or it can also indicate an increase in the amount of analyte.

With more detail, identifying an aggregate can include imaging the sample to create a sample image. The sample can include a plurality of particles of the first-type and/or a plurality of particles of a second-type. Each pixel of the sample image can be assigned a color-scale value based on the intensity of a color in the pixel. A color-scale can be a gray-scale; however, other colors can be used. A distribution of the color-scale values assigned to each pixel in the sample image can be calculated. As discussed above, the distribution can be used to determine the presence of an aggregate, and the presence of an aggregate can indicate the presence of the target analyte.

A method of identifying an aggregate can also include imaging a control. The control can be a negative control. The control can be a sample or blank in the absence of the analyte and/or the particles. Imaging a control can create a control image. Each pixel of the control image can be assigned a color-scale value based on the intensity of a color in the pixel. A distribution of the color-scale values assigned to each pixel in the control image can be calculated. The distribution of the color-scale values assigned to the pixels in the sample image can be compared to the distribution of the color-scale values assigned to the pixels in the control image. A wider distribution of the color-scale values assigned to the pixels in the sample image than the distribution of the color-scale values assigned to the pixels in the control image can indicate the presence of an aggregate, and the presence of an aggregate can indicate the presence of the target analyte. The distribution of the color-scale values assigned to the pixels in the control image can also be used as a baseline to make corrections to a measurement of the degree of aggregation.

A similar process for a positive control for aggregation can also be performed. A sample including the target analyte would be expected to have a distribution of the color-scale values similar to that of the positive control.

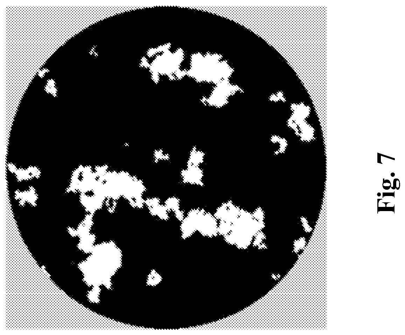

In a second approach, each pixel of the sample image is assigned a binary-value. For example, the pixel can be assigned 0 or 1, plus or minus, or black or white (Fig. 7). To facilitate the assignment of a binary value, the image can be processed as a binary image (e.g., a black and white image). The percentage of the area of the image occupied each binary value can be calculated. As one of the two binary values indicates the presence of an aggregate (positive binary value), the presence of a pixel with that binary value can indicate the presence of an aggregate. The percentage of pixels with the positive binary value can indicate the degree of aggregation.

More specifically, identifying an aggregate can include imaging the sample including a plurality of particles of a first-type and/or a plurality of particles of a second-type to create a sample image. A binary value can be assigned to each pixel of the sample image based on the intensity of a color in the pixel. As a non-limiting example, on a color-scale where 0 is black and 1 is white, each pixel with a color-scale value of 0.00 - 0.49 can be assigned the binary value of 0 and each pixel with a color-scale value of 0.50 - 1.00 can be assigned the binary value of 1. A first binary value (the positive binary value) can indicate the presence of an aggregate. The number of pixels in the sample image assigned to a first binary value can be calculated. If one or more pixel is assigned the first binary value, it can indicate the presence of an aggregate.

Identifying an aggregate can also include imaging a control to create a control image. The control can be a negative control. The control can be a sample or blank in the absence of the analyte and/or the particles. A binary value can be assigned to each pixel of the control image based on the intensity of a color in the pixel, as discussed above. The number of pixels in the control image assigned to the first binary value can be calculated. The number of pixels in the sample image assigned to the first binary value can be compared with the number of pixels in the control image assigned to the first binary value. The presence of an aggregate can be indicated when the number of pixels in the sample image assigned to the first binary value is greater than the number of pixels in the control image assigned to the first binary value. The number of pixels in the control image assigned to the first binary value can also be used as a baseline to make corrections to the calculation for the sample image.

Identifying an aggregate can include quantifying the degree of aggregation using the assigned binary values. Quantifying the degree of aggregation can include calculating the percentage of pixels in the image of the sample assigned to the first binary value. Again, the percentage of pixels in the control image assigned to the first binary value can also be used as a baseline to make corrections to the calculation for the sample image.

A positive control for aggregation can also be used with this approach.

The methods described can be performed using microfluidics. For example, the methods can be performed in a well or cell. A well can have a diameter less than 10 mm, less than 8mm, less than 6mm, less than 5mm, less than 4mm, less than 3mm, less than 2mm, less than 1.5 mm or less than 1 mm. A well can be part of an array. Each well of an array can include a particle. In some circumstances, an array can include a plurality of wells.

A well can be a divot, a tube, a tray, a well or a similar compartment for suitable for containing a sample. A plurality of wells can be in a pattern on a substrate. A pattern can include concentric circles, a spiral, a row, a column or a grid.

A cell can be a column. A cell can have a width that is less 1100 µm, less than 1000 µm, less than 800 µm, less than 600 µm, less than 500 µm, less than 250 µm, less than 100 µm, less than 50 µm, less than 25 µm, or less than 10 µm. A cell can have a height that is less than 1100 µm, less than 1000 µm, less than 800 µm, less than 600 µm, less than 500 µm, less than 250 µm, less than 100 µm, less than 50 µm, less than 25 µm, or less than 10 µm. A cell can include a filter. In some cases, a cell can have a width or a height that is theoretical. A filter can have a pore that is less than 10 µm, less than 5 µm, less than 1 µm, less than 0.5 µm, less than 0.1 µm, less than 0.05 µm, less than 0.01 µm, less than 0.005 µm, or less than 0.001 µm.

In another method, based on agglutination in microchannels, a simple system for quantitative sensing of a target analyte can be used. Functionalized microbeads and analyte with no prior incubation can be flowed in droplets (for example, ∼2µl) through a thin silicone tube filled with mineral oil. High flow rate can be used. For example, the flow rate can be 150µl/min. Hydrodynamic forces alone can produce a highly efficient mixing of the beads within the droplet, without the need of complex mixing structures or magnetic actuation. The setup allows rapid observation of agglutination (<2 min), which can be quantified using image analysis, and has potential application to high-throughput analysis.

Agglutination assays are typically performed manually on cards and are qualitative or semi-quantitative. Performing them in a microfluidic device has advantages such as increasing their sensitivity and speed while reducing the required sample volume. See, for example, "Improving agglutination tests by working in microfluidic channels," G. Degre, E. Brunet, A. Dodge, and P. Tabeling, Lab on a chip, 5, pp. 691-4, (2005). Antibody and protein agglutination assays in plugs have been demonstrated previously using microfluidic devices; however, these studies have been done at low flow rates and use either winding channels to enhance the mixing of the agglutination components (5.5µl/min) or a concentrated magnetic field (up to 1µl/min), and require previous incubation. See, for example, "Improving agglutination tests by working in microfluidic channels," G. Degre, E. Brunet, A. Dodge, and P. Tabeling, Lab on a chip, 5, pp. 691-4, (2005); "ABO, D blood typing and subtyping using plug-based microfluidics," T. R. Kline, M. K. Runyon, M. Pothiawala, and R. F. Ismagilov, Analytical chemistry,80, pp. 6190-7, (2008). However, the system in Kline uses winding channels to enhance the mixing of droplets flowing at 5.5 µl/min; and Teste requires external magnetic actuation (magnetic tweezers) during data extraction, and runs at low flow rates (up to 1 µl/min), and requires previous incubation.

Simple droplet velocity control can be used to affect the circulation and aggregation of non-functionalized beads. The method described herein takes advantage of the efficient mixing generated within a droplet at high velocities to perform agglutination in a simple and rapid way, without the need for magnetic fields and at flow rates that allow for much higher throughput in the future.

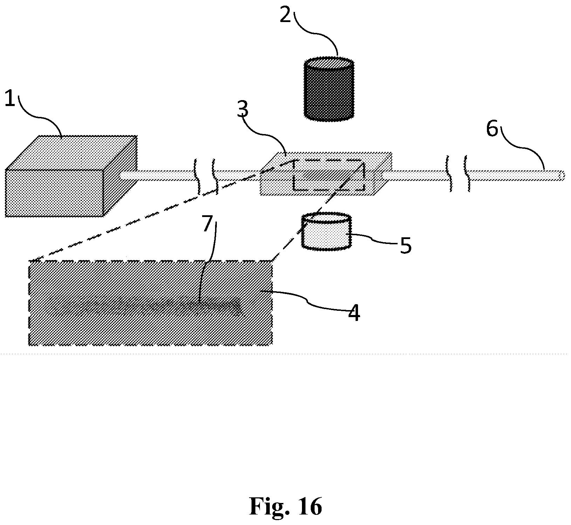

FIG. 16 is a schematic representation of the experimental setup for in-droplet incubation and quantification of agglutination assays. 1 is a syringe pump, 2 is a camera, 3 is a PDMS block, 4 is mineral oil, 5 is a lamp, 6 is a tubing (such as 0.51 mm ID silicone tubing), and 7 are droplets (such as 2 µL droplet). The tubing can be transparent. The tubing can be a silicone tubing, peek tubing, teflon tubing, tygon tubing, and so on. Silicone tube, such as A150 cm silicon tube, can be filled with mineral oil and driven by a syringe pump. Droplets containing a bead and analyte mixture can be inserted into the tube at the open end. Images can be taken with a camera placed over a PDMS block (used for refraction index matching with the tube) at mid-length of the tube.

The agglutination of both protein and DNA based agglutination assays can be reliably quantified. At high analyte concentrations the "hook effect", characteristic of agglutination assays, can be observed. Increasing the concentration of microbeads can increase the range and degree of agglutination, with DNA tests showing an even wider range. These measurements can be performed rapidly and at high flow rates, without the need for incubation, complex mixing structures or magnetic actuation.

High throughput and short sample-to-answer times are desirable features in diagnostic and agglutination assays. The droplet system requires no prior incubation of the agglutination mixture, which combined with its highly efficient mixing allows a rapid readout of signal after insertion of the sample. Due to its setup and high flow rates, the system can be used for high volume series testing. This system can also be used for more complex assays that may require thermal cycling, as the tubing can be arranged in loops over heating or cooling platforms.

The assays are performed in a simple silicone tube at high flow rates, without the need of complex mixing structures or external magnetic actuation. The higher flow rates produce an efficient mixing which allows the samples to be read within two minutes after insertion of the system, while commercially available products take at least five times longer. The flow rates of the system are 30-50 times higher than other similar systems previously published, which indicate a capability of at least 10 times higher throughput of results.

### APPLICATIONS

The methods described, and devices performing these methods, can have a broad applicability. The methods and devices can provide the basis for a flexible point-of-care (POC) assay. The methods can be used to detect the presence of atoms and molecules, such as proteins or specific DNA sequences, at room temperature in just a few minutes. This can open possibilities to develop POC or home use based diagnostics based on the method.

The method can be applicable to disease diagnosis, pharmagenomics, pathogen detection and/or agro-food testing, or environmental testing. Besides protein and DNA detection, the methods can be used to detect ions (such as heavy metal ions, indicating pollution), organic chemicals (pesticides, herbicides) and other targets. Functionalization of microbeads by recognizing molecule can allow combinations leading to agglutination and visible signal of target detection.

The described methods can also be utilized in the personalized medicine. DNA sequences can differ from individual to individual. It can be important to determine the DNA variations (differences). DNA variations can be useful for biomarker research, prediction of genomic predispositions and more. The methods can be used in research centers, hospitals and even by individuals. The assay can be run on small chips simply by connecting the chips to the computer or hand held device through USB or other connection. Microfluidics can be applied for the diagnostics in clinics, veterinary, industry, forensics and other areas of life.

The methods can be useful for clinical diagnostics of bacterial and viral infectious diseases, and for the detection of antibiotic resistant bacteria. In personalized medicine, the patients' health can be effectively managed based on individual patients specific characteristics, inclusive the genetic variations. Using the described methods, genetic testing can be possible in pharmagenomics or for the determination of genetic predisposition. In oncology, the methods can be useful for diagnostics of various kinds of cancers. In the veterinary field, the health and safety of animals can be assessed. In industrial diagnostics, food and water safety can be examined, inclusive for the genetically modified organisms and food. In environmental monitoring, the methods can be used to check the soil, water and air quality for any DNA-containing virus or bacteria. In case of any bio-related infections, a device performing the methods described can perform real-time monitoring and detection of harmful particles. Additionally, the methods described can be useful for the research in pharma/biotech and in academia or for forensics, such as for DNA profiling. Further, the two- or three-component assay can be suitable to analyze the environmental samples. Specific atoms, ions and molecules can be identified and quantified.

In summary, the methods described, and devices performing these methods can be used for the detection of infectious diseases, inclusive of third world infections. They can be used as emergency tests or home tests, both at doctor office for screening or as decentralized hospital test. They can be used for water testing, agro-food testing, and can be useful both in forensics and military.

Practical examples of the use of the methods described, and devices performing these methods can include:
- Detection and identification of virus particles during the flu season. After loading a tiny amount of saliva and running an assay connected to the personal computer (or to the hand phone), the result can quickly report whether the particular condition is due to common flu or because of other causes (different virus, etc.).
- Monitoring of chronic disease (e.g. diabetes or cardiovascular diseases). Detection of the presence of particular molecules in the body can inform and instruct the patient when to take particular kind of medicine. Also, the healing process following, for example, a viral infection like the flu, can be monitored over time.
- During the sickness, assay can inform the patient the best suitable time and amount to take a medicine that may depend on the presence of specific molecules in the body.
- Detection of a predisposition to cancer and other diseases and possible disease development can be monitored over the life time. One advantage, here, can be that not only can DNA be monitored by various assays, but also transcriptome changes (RNA) can be detected.
- Testing food for the origin (pork, cow, etc.) in the agriculture or fishery industry. Also, genetically modified food can be easily tested over short period of time using only a small amount of food sample.
- Single-nucleotide polymorphism-based genotyping of living organisms (fish, livestock, etc.) can be performed.
- Livestock can be tested for the genetic markers to determine which animals will best develop the desired traits.
- In the immigration or police department, the forensic analysis can be performed to screen people apprehended for potential crimes (identify human DNA in few minutes with a lower cost for the test).
- Also, the test can be used to screen the visitors to the country at arrival for past crimes or potential diseases or infections, like tuberculosis.

### EXAMPLES

### Two-Component Hybridization

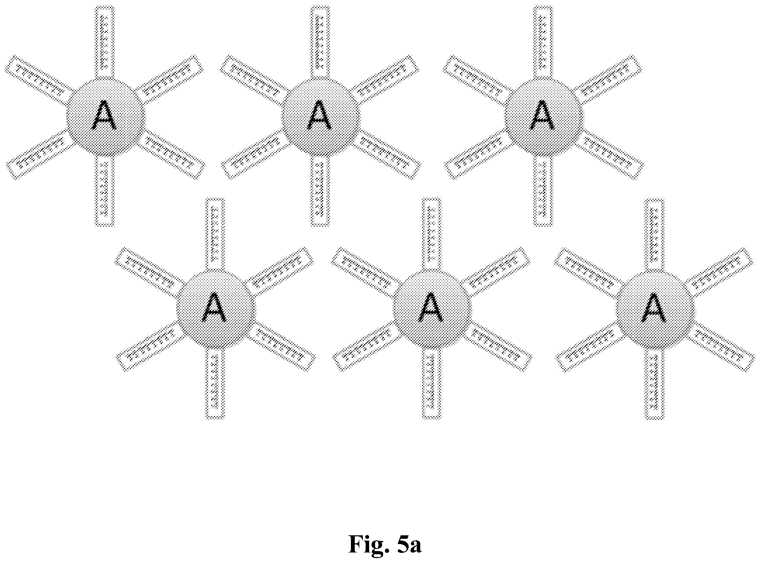

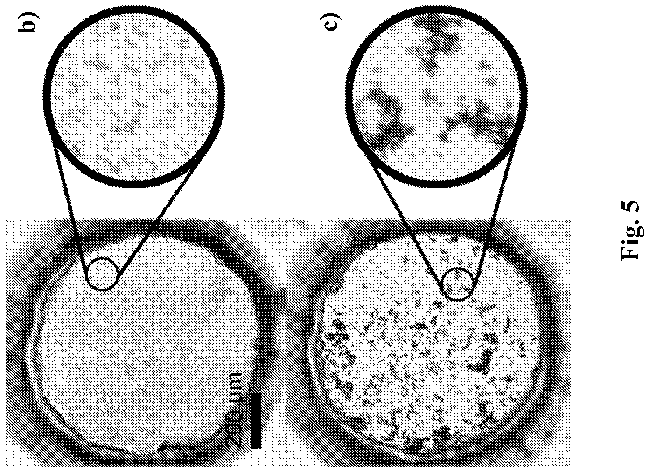

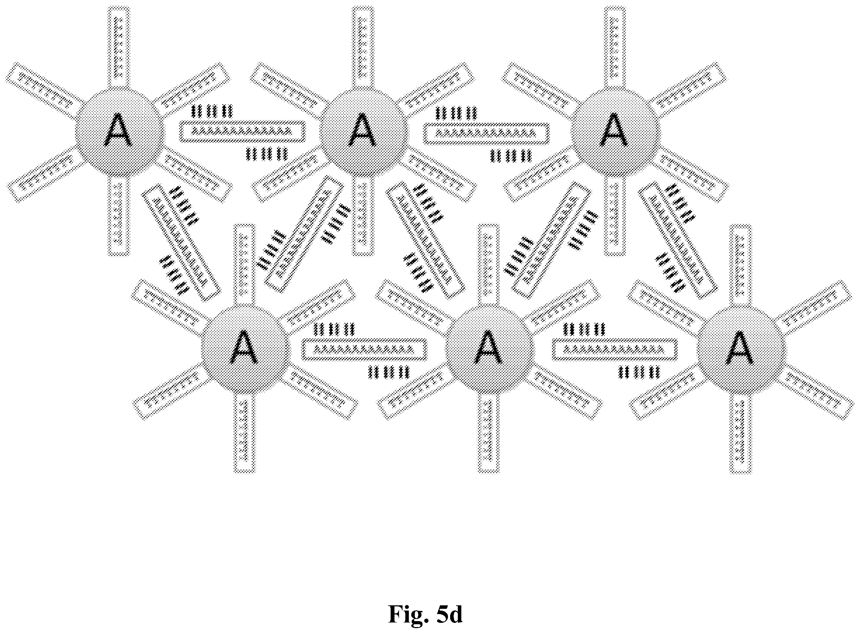

A two-component hybridization assay using microbeads was performed (Fig. 5). A schematic of the microbeads is shown in Fig. 5a prior to being exposed to the target analyte. The microbeads were added to a sample in 800 µm diameter wells (Fig. 5b and 5c). The wells were made by CO₂ laser cutting bio-compatible adhesive tape (BIO-RAD, Microseal®B, Adhesive Sealer) and bonding the tape to the microscope glass slide. Imaging was done using Canon photo camera mounted on Zeiss microscope with 10x magnification objective. Fig. 5b shows an example of a sample with no agglutination present (top). Fig. 5c shows a sample with agglutination (bottom). Fig. 5c is a schematic of an aggregate, including the microbeads following exposure to the target analyte.

### Detection of Aggregates

A script in Matlab programming environment was written for image analysis to quantify the level of agglutination present, by two different methods. The first method (the color-scale or grayscale method), illustrated in Figure 2, was based on the distribution of the image's grayscale values (ranging from 0 for black to 1 for white). Using this approach, a negative sample can tend to have a narrow distribution around the middle of the grayscale (low standard deviations), while a positive sample can tend to have a wide distribution (higher standard deviation). Therefore, this approach can be used to determine both the presence of aggregates and the degree of agglutination.

As shown in Fig. 6, the grayscale method can be used to quantify the level of agglutination present. Fig. 6a shows grayscale images of a negative test and Fig. 6c shows a positive test. The histogram in Fig. 6b corresponds with the image of Fig. 6a, and the histogram in Fig. 6d corresponds with the image of Fig. 6c.

A second approach (the binary method), illustrated in Figure 7, was based on processing of a binary image (black and white values only). As shown in Fig. 7, the binary method can be used to quantify the level of agglutination present. Fig. 7 shows a binary image of a positive test. The percentage of the image's area occupied by agglutinated particles can be calculated and can also be used to determine both the presence of aggregates and the degree of agglutination.

### Comparison of Two-Component and Three-Component Assays

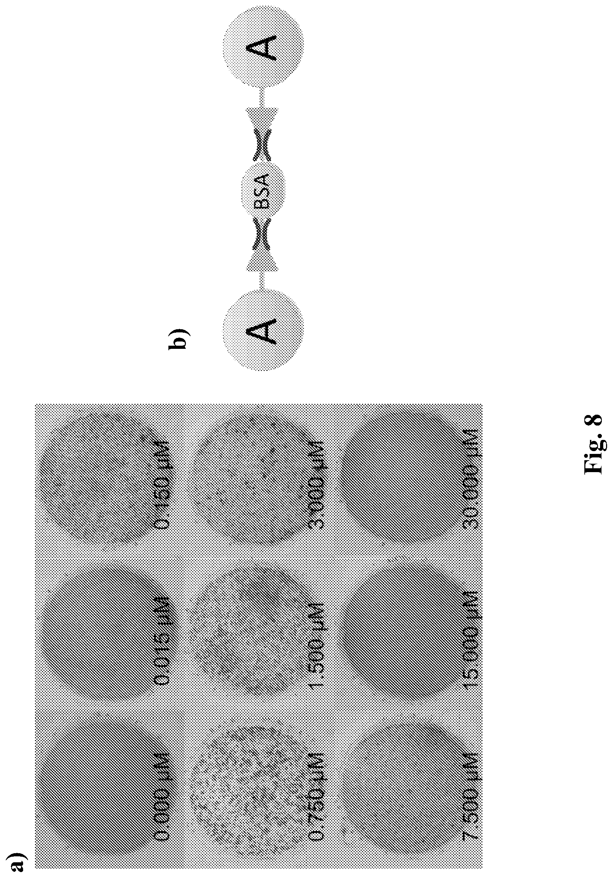

The two-component bead agglutination assay (specified as A + B + A) was performed using streptavidin-coupled beads ("A") and biotinylated Bovine Serum Albumin (BSA) protein ("B"). The (A + B + A) assay is schematically represented in (Fig. 8b). The target concentration of biotinylated BSA protein was titrated from 0 to 30 µM final concentration (Fig. 8a)

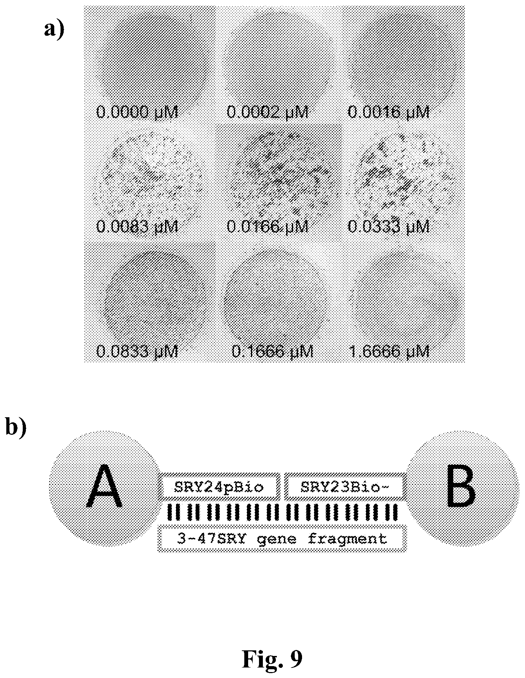

A three-component assay based on DNA hybridization (A + B + C) was performed using two different pools of beads functionalized with complimentary oligonucleotides (A or B) (Fig. 9b). Target DNA (3-47SRY) concentration was titrated by mixing with oligo SRY24pBio, SRY23Bio-functionalized microbeads in a solution (A + B + C) (Fig. 9a). DNA sequences specific to male sex-determining region Y (SRY) gene (Table 1 below) were successfully tested, enabling the hybridization and following agglutination to be performed at room temperature (Fig. 9a). The DNA target concentration was titrated from 0 to 1.67 µM final concentration of 3-47SRY oligonucleotide. The assay was performed using SRY gene detection assay, as an example; however, the assay is suitable to detect any DNA sequence.

**Table 1. Oligonucleotides used for hybridization assays. 5'- and 3'- end modifications are indicated.**

| Oligo name | 5'-modification | Sequence | 3'-modification |
|---|---|---|---|
| SRY24pBio | phosphate | GCGAAGATGCTGCCGAAGA ATTGC | Biotin |
| SRY23Bio- | Biotin | ATAAGTATCGACCTCGTCGG AAG | none |
| 3-47SRY | none | GCAATTCTTCGGCAGCATCT TCGCcttccgacgaggtcgatacttat | none |

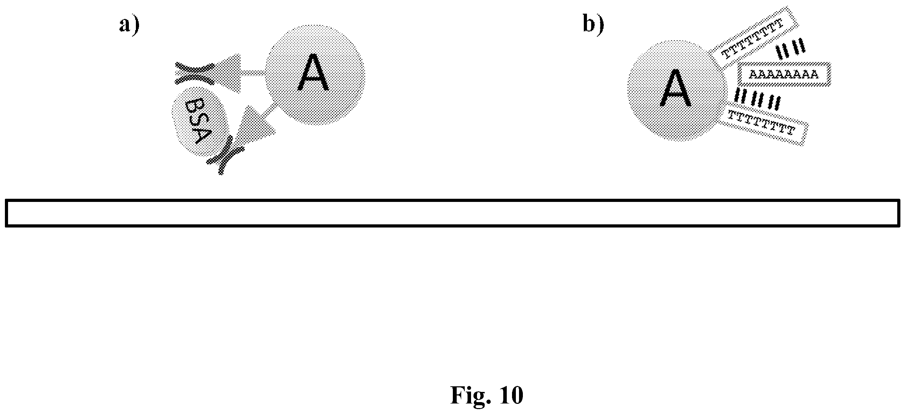

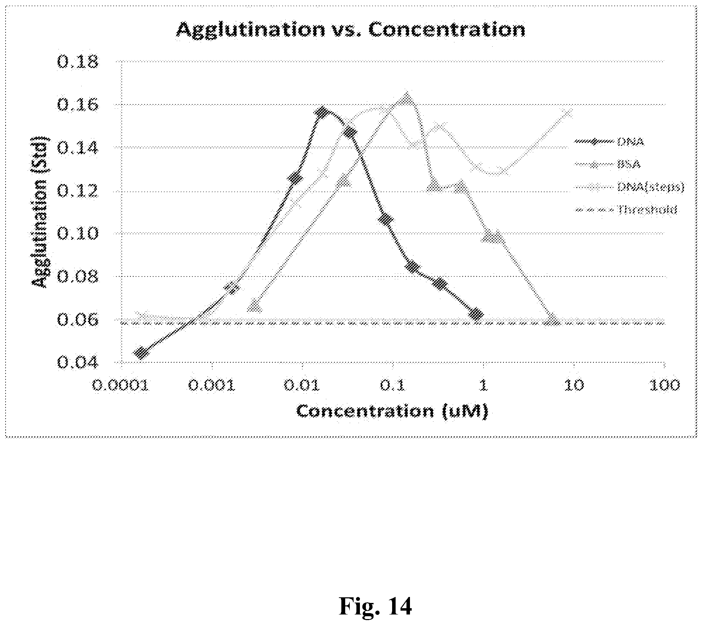

For protein (BSA) based assays (A + B + A), concentrations as low as 0.150 µM of biotinylated BSA could be detected by agglutination, whereas for DNA based assays (A + B + C), ssDNA with concentration as low as 0.0167 µM can be detected (Fig. 9a). The difference may be due to the linking agent in the two-component case being able to link two sites on the same bead and thus self-terminate, wasting analyte (See, for example, Figs. 10a and 10b). The non-self-terminating ends of the three-component (A + B + C) assay allows detection at an order of magnitude lower concentration of target molecule in comparison to the assay with self-terminating possibility (Figs. 9b and 14).

The two-component assay can have limitations. As shown in Fig. 10, the target (biotinylated BSA or oligo) in the two-component assay can link two sites on same bead. This can hinder agglutination and can result in higher concentrations of analyte required to produce agglutination.

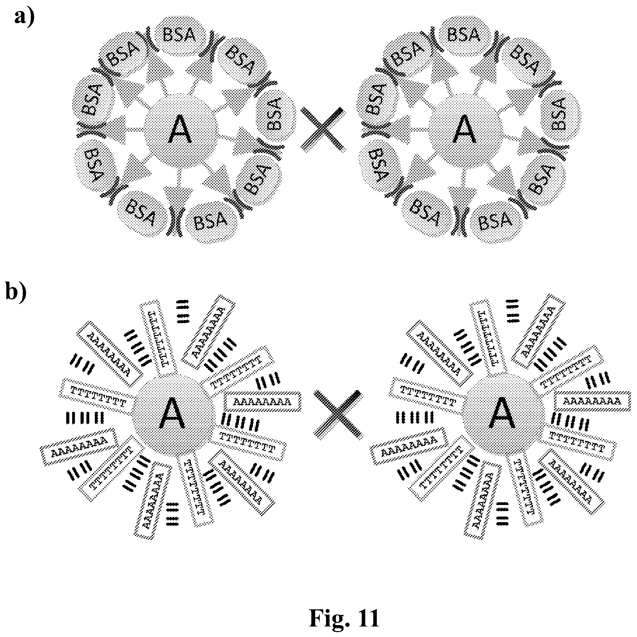

As shown in more detail, the target (biotinylated BSA or oligonucleotide) in the two-component assay can link two sites on same bead and can hinder agglutination (Figs. 11a and 11b). This can result in higher concentrations of analyte being required to produce agglutination. As shown in Fig. 11a, BSA protein can saturate active sites on same bead, thus preventing the bead agglutination. DNA based site saturation (A + B + A) on same bead can have a similar problem, where the beads cannot agglutinate because there are no active sites suitable for agglutination.

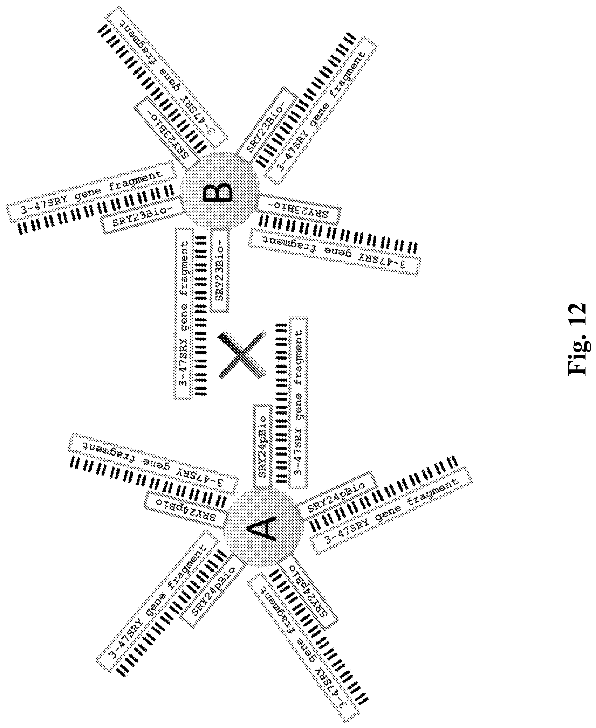

Further, Fig. 12 illustrates that simultaneous addition of more one particle (non-stepped assays) can also be prone to active site saturation and loss of agglutination at high analyte concentrations. A schematic of active site saturation is shown in Fig. 12. The protruding single stranded DNA may not hybridize because it is not complementary, as the sequence on another bead is the same.

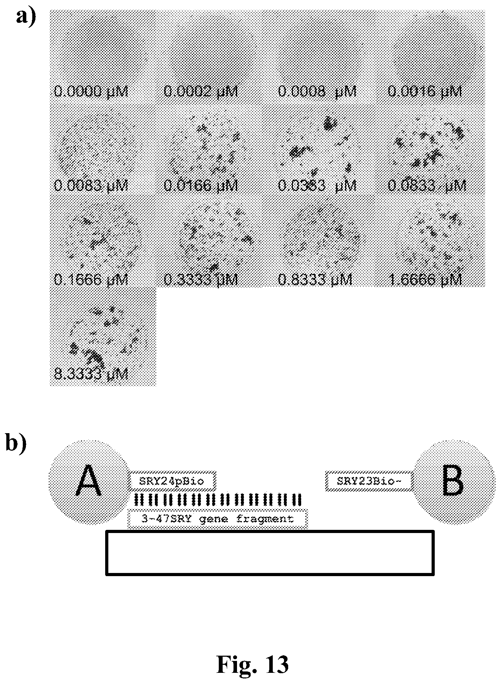

A stepped three-component hybridization assay ((A + C) + B) (Figs. 2 and 13b). Generally, in the stepped assay, one kind of functionalized bead (A) was used to bind the target (in this case hybridization with DNA (C)). The unbound fraction of the target was then washed away. Other beads (B, functionalized with different molecules) were then introduced to the construct made of target bound to beads (A + C). The B-type beads were not saturated with analyte in this scheme, and thus, readily agglomerated with the saturated A-type beads and maintained high agglutination signal, even at very high analyte concentrations.

SRY gene hybridization was used for a stepped three-component hybridization assay. Beads functionalized with oligo SRY24pBio were mixed with various concentrations of target DNA (3-47SRY). After hybridization and washing away non-hybridized DNA, SRY23Bio- functionalized microbeads were introduced ((A + C) + B), followed by agglutination. Fig. 13b shows each of the assays labeled with its µM final concentration of (3-47SRY) oligonucleotide.

The stepped three-component assay was an order of magnitude more sensitive than two-component assay and maintained positive signal at high analyte levels (Fig. 14). The agglutination strength versus micromolar final analyte concentration is depicted in Fig. 14. It should be noted that the stepped assay concept can be applied to the two-component assay, as well, to improve its measurement range at high concentrations.

### Ligation Based Agglutination Assays

Ligation based agglutination (LBA) can be performed to ligate the bridging DNA from bead A to bead B. Ligation reaction can occur only if oligonucleotides are in close proximity and are complementary to the recognized DNA (Fig. 4). The covalent bond can be formed between DNA, and the beads may not be separated without destroying the covalent bond. As shown in Fig. 4, ligase can seal the gap between two oligonucleotides forming the covalent bond. The two types of beads (A and B) can be bound through DNA.

Thermal heating may have no or minimal impact on the agglutinated beads because of ligated DNA. Cycling the temperature (cycling ligation based agglutination assay, indicated as C-LBA) can allow the release of the target DNA to be detected, and can allow the target DNA to anneal to new oligonucleotide strands functionalized to beads A and B (Fig. 3). This ability to reuse the analyte through thermal cycling should further increase the sensitivity of the test through an intrinsic amplification. Ligation can form covalent bonds, strengthening the agglutination (Fig. 3). In C-LBA, the detection level should be even lower than the described two or three-component assays.

A single temperature amplification of the signal has also been performed using enzymes that amplify RNA or DNA at single temperature (i.e. no need to cycle the temperature). As more DNA means more analyte, the agglutination assay can be faster and the signal can be stronger.

### Detection of Aggregates

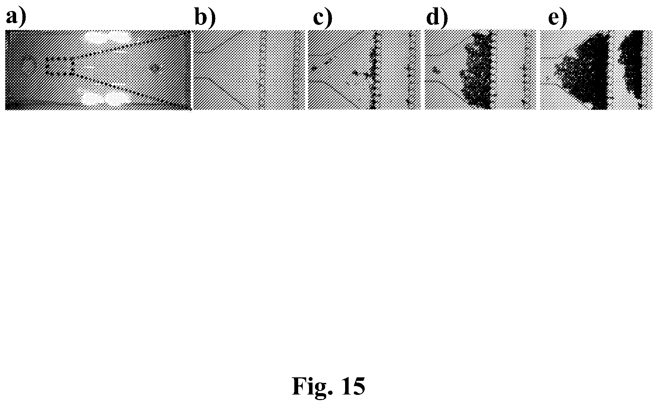

The agglutination was also demonstrated in microfluidic channels in a PDMS microfluidic chip. Non-agglutinated beads can move freely through the filter made of 50 µm high columns spaced by 10 µm. However, when agglutinates are formed, they are held by row of columns (Fig. 15). The agglutinated beads can be seen by naked eye. The PDMS microfluidic device can consist of a reaction chamber, where beads are pipetted, the filter with 10 µm spaced 50 µm high columns and a waste output, where negative pressure can be applied. As shown, the flow direction was from left to right. Fig. 15a shows a view of PDMS microfluidic chip. The marked area is magnified. Non-bound beads were able to flow freely through the columns in microfluidic device (Fig. 15b). Agglutinated beads preferentially stuck to first row of columns (Fig. 15c). The agglutinated beads were visible by naked eye (Fig. 15d). In some cases, higher flow rates moved some agglutinated beads through columns; however, they were captured by next row of columns (Fig. 15e).

The detection limit can be further improved using other detection methods, for example, measuring resistance of the sample. For example, a microfluidic Coulter Counter can be used. The Coulter Counter is a class of devices used to count and take accurate measurements of the size of particles suspended in a fluid using electric resistance of a fluid. For the methods described herein, this type of device can be applied to measure the size distribution of the agglutinates, which will allow for highly accurate quantification of the agglutination assay with fewer beads and less analyte than even the current image analysis method. Additional measurements can be performed without any optical system.

The results can be summarized as:
- A three-component assay has been demonstrated, which can have increased sensitivity over two-component assays. This increase can be up to an order of magnitude improvement in sensitivity.
- The three-component assay has increased measurement range. The saturation effect has been removed or minimized by use of the stepped three-component assay. The saturation effect can cause loss of signal by using a stepped assay.
   - Two kinds of image analysis methods were developed (indicated as grey and binary methods). Both were suitable for automated analysis and quantification of agglutinated beads.
   - Coulter Counter based detection has been proposed for greater sensitivity and better quantification of the agglutination results.
   - Ligation based agglutination and Cycling Ligation based agglutination assays can be used to push the detection limit even further. Temperature cycling and single temperature versions of the Cycling Ligation based agglutination assay have been successfully performed.
   - An assay using aptamers for the recognition can be used for the recognition of virtually any of the target.
   - The assays can be performed at room temperature, and are fast (measurement result in 5 minutes).
   - The two- and three-component assays can be used to analyze environmental samples. Specific atoms, ions and molecules can be identified and quantified

This technology has demonstrated differences from prior approaches in the following ways:
- The three-component assay (A + B + C) can solve the linker self-termination issue allowing an order of magnitude increase in sensitivity over two-component assays.
- The stepped version of the three-component assay ((A + C) + B) can solve the issue with probe site saturation thus enabling a wider range of detection. This stepped concept can also be applied to two-component assays to increase detection range.
- Detection of the agglutinated beads with the naked eye can be performed by trapping in microfluidic channels.
- Image analysis for quantification of agglutination can be performed. The image analysis can be performed automatically.
- An assay using aptamers for the recognition can be used for binding of virtually any of the target.
- Coulter Counter based detection can be used for greater sensitivity and better quantification of the agglutination results.
- LBA and C-LBA assays can push the detection limit even further. The C-LBA assay can be performed quickly using a single temperature amplification.

### A Simple System For In-Droplet Incubation And Quantification Of Agglutination Assays

A droplet of agglutination mixture (∼2µl), formed by functionalized microbeads and analyte, was pipetted into a container with mineral oil. Droplets and analyte mixture then were flowed through a silicone tube (150µl/min) using a syringe pump (FIG. 16).

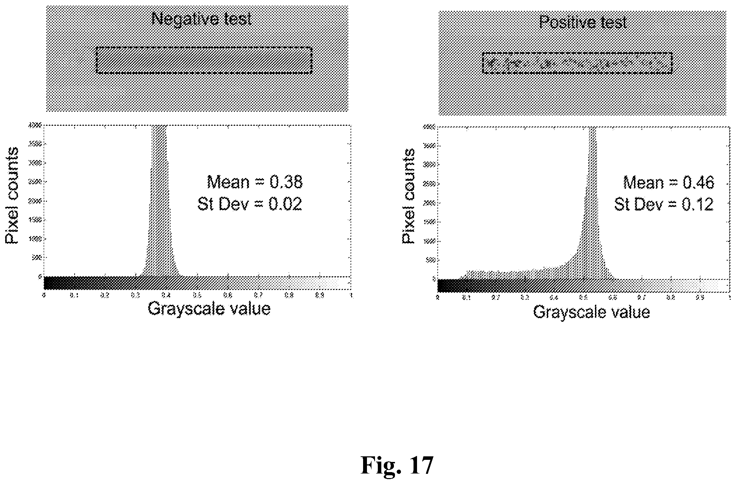

Using a Canon EOS 5D MarkII camera and 1-5x macro lens, videos were taken of the passing droplets; images were extracted and processed with a script in Matlab. Agglutination was quantified by the distribution of the image's grayscale values, FIG. 17. FIG. 17 shows the representation of the quantification of agglutination. The histogram of the image at the cropped region will have a narrow distribution (low standard deviation, σ) in negative tests (left), and a wide distribution (higher standard deviation, σ) in positive tests (right).

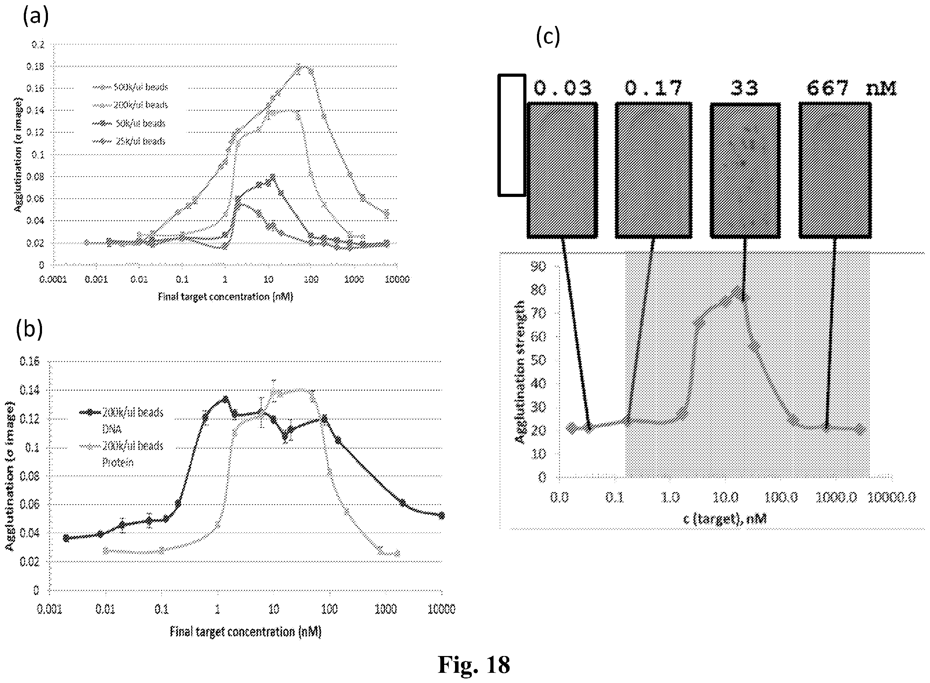

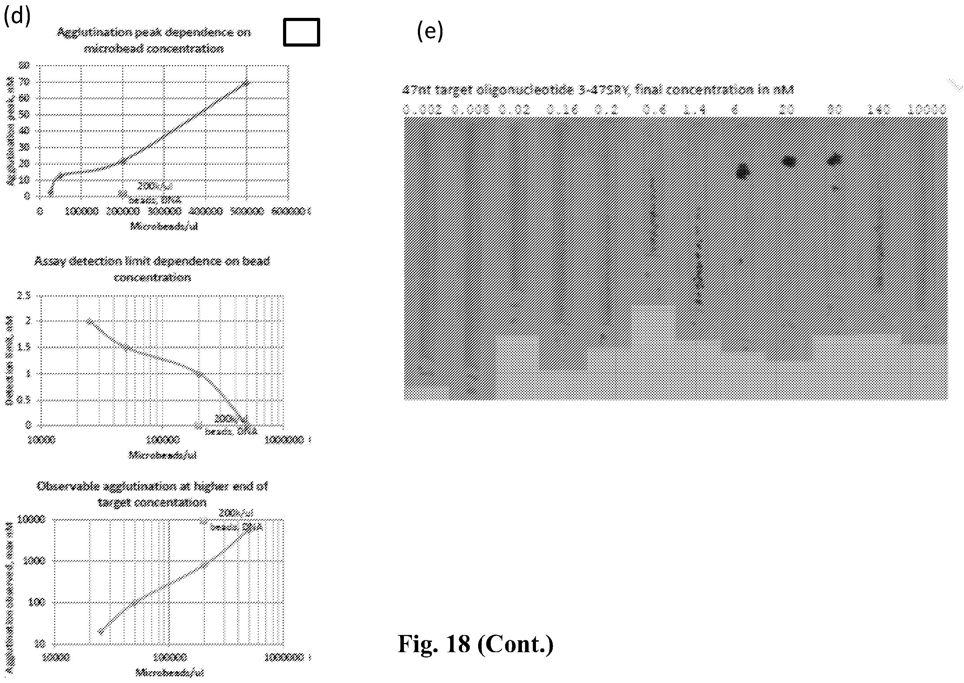

The effect of varying both the microbead and analyte concentrations was studied. Dynabeads M-270 streptavidin beads in 1x PBS/1% BSA buffer were varied from 25k/µl to 500k/µl. The target analyte composed of biotinylated BSA (Sigma) was tested at a wide range of concentrations, from 1pM to 40µM. Silicone oil AR20 (Sigma) with 1% (w/w) nonionic surfactant Span 80 was used as the carrier fluid. For DNA testing, beads were functionalized with oligonucleotides to match a target 47 base pair section of the SRY gene. Results are shown in FIGS. 18(a) and (b). FIGS. 18(a) and (b) are curves showing agglutination strength, each point is the average measurement of 5 photographs of the droplet, error bars are the standard deviation. FIG. 18(a) shows effect of varying the concentration of the target analyte and beads in a biotin-streptavidin assay. FIG. 18(b) shows agglutination at 200k beads/µl for biotin-streptavidin and DNA tests. FIG. 18(c) is another example showing dependence of agglutination strength on target concentration. FIG. 18(d) shows dependence of agglutination peak, assay detection limit and observable agglutination on microbeads concentration. FIG. 18(e) shows images at different concentrations. The following table 1 lists examples of agglutination peak, lower detection, and range of detection for different assays at different beads concentrations.

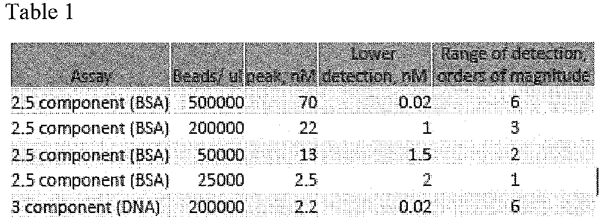

## Claims

1. A method for detecting the presence of an analyte in a sample, comprising:
adding a plurality of microparticles of a first-type to the sample, wherein each microparticle of the first-type includes a first binding partner configured to interact with at least a first portion of the analyte;
adding a plurality of microparticles of a second-type to the sample, wherein each microparticle of the second-type includes a second binding partner configured to interact with at least a second portion of the analyte, the first portion of the analyte being different from the second portion of the analyte; and
identifying an aggregate including at least one microparticle of the first-type, at least one microparticle of the second-type and the analyte, wherein identifying an aggregate includes quantifying the degree of aggregation, and wherein the aggregate indicates the presence of the analyte.

2. The method of claim 1, wherein the plurality of microparticles of the second-type is added to the sample at the same time as the plurality of microparticles of the first-type is added to the sample.

3. The method of claim 1, wherein the plurality of microparticles of the second-type is added to the sample after the plurality of microparticles of the first-type is added to the sample.

4. The method of claim 1 or 3, wherein the plurality of microparticles of the second-type is added to the sample less than 10 minutes after the plurality of microparticles of the first-type is added to the sample, such as wherein the plurality of microparticles of the second-type is added to the sample less than 5 minutes after the plurality of microparticles of the first-type is added to the sample.

5. The method of any one of claims 1-4, wherein the first portion of the analyte and the second portion of the analyte have a common region.

6. The method of any one of claims 1-4, wherein the first portion of the analyte and the second portion of the analyte are independent.

7. The method of any one of claims 1-6, wherein the analyte is a nucleic acid, a polypeptide or a polysaccharide.

8. The method of any one of claims 1-7, wherein the first binding partner is a carbohydrate, a nucleic acid or a polypeptide.

9. The method of any one of claims 1-8, wherein the second binding partner is a nucleic acid or a polypeptide.

10. The method of any one of claims 1-9, wherein the first binding partner is associated with the at least one microparticle of the first-type through a covalent, ionic, van der Waals, dipolar or hydrogen bond, such as through a covalent or ionic bond.

11. The method of any one of claims 1-10, wherein the second binding partner is associated with the at least one microparticle of the second-type through a covalent, ionic, van der Waals, dipolar or hydrogen bond, such as through a covalent or ionic bond.

12. The method of any one of claims 1-11, wherein identifying an aggregate includes:
imaging a control in the absence of the analyte to create a control image;
imaging the sample including the plurality of microparticles of the first-type and the plurality of microparticles of the second-type to create a sample image;
assigning a color-scale value to each pixel of the control image based on the intensity of a color in the pixel;
assigning a color-scale value to each pixel of the sample image based on the intensity of a color in the pixel;
calculating a distribution of the color-scale values assigned to the pixels in the sample image;
calculating a distribution of the color-scale values assigned to the pixels in the control image; and
comparing the distribution of the color-scale values assigned to the pixels in the sample image to the distribution of the color-scale values assigned to the pixels in the control image, wherein a wider distribution of the color-scale values assigned to the pixels in the sample image than the distribution of the color-scale values assigned to the pixels in the control image indicates the presence of an aggregate.

13. The method of claim 12, further comprising flowing at least one droplet of an agglutination mixture through a channel, such as further comprising quantifying the agglutination by the distribution of the image's grayscale values.

14. The method of claim 13, wherein the agglutination mixture is formed by functionalized microbeads and the analyte, and preferably wherein the flow rate is between 25 µL/min and 500 µL/min.

15. The method of any one of claims 1-11, further comprising measuring resistance of the agglutination mixture.

16. The method of any one of claims 1-11, wherein identifying an aggregate includes:
imaging the sample including the plurality of microparticles of the first-type and the plurality of microparticles of the second-type to create a sample image;
assigning a binary value to each pixel of the sample image based on the intensity of a color in the pixel; and
calculating the number of pixels in the sample image assigned to a first binary value, wherein the assignment of the first binary value to any one pixel indicates the presence of an aggregate.

17. The method of claim 16, wherein identifying an aggregate includes:
imaging a control in the absence of the analyte to create a control image;
assigning a binary value to each pixel of the control image based on the intensity of a color in the pixel;
calculating the number of pixels in the control image assigned to the first binary value; and
comparing the number of pixels in the sample image assigned to the first binary value with the number of pixels in the control image assigned to the first binary value, wherein the presence of an aggregate is indicated when the number of pixels in the sample image assigned to the first binary value is greater than the number of pixels in the control image assigned to the first binary value.

18. The method of claims 1 to 17, wherein quantifying the degree of aggregation includes calculating the percentage of pixels in the image of the sample assigned to the first binary value.

## Patentansprüche

1. Verfahren zum Nachweisen des Vorhandenseins eines Analyten in einer Probe, das umfasst:
Hinzufügen einer Mehrzahl von Mikropartikeln eines ersten Typs zur Probe, wobei jeder Mikropartikel des ersten Typs einen ersten Bindungspartner umfasst, der so konfiguriert ist, dass er zumindest mit einem ersten Abschnitt des Analyten interagiert;
Hinzufügen einer Mehrzahl von Mikropartikeln eines zweiten Typs zur Probe, wobei jeder Mikropartikel des zweiten Typs einen zweiten Bindungspartner umfasst, der so konfiguriert ist, dass er mit zumindest einem zweiten Abschnitt des Analyten interagiert, wobei der ersten Abschnitt des Analyten sich vom zweiten Abschnitt des Analyten unterscheidet; und
Identifizieren eines Aggregats, das zumindest einen Mikropartikel des ersten Typs, zumindest einen Mikropartikel des zweiten Typs und den Analyten umfasst, wobei das Identifizieren eines Aggregats das Quantifizieren des Aggregationsgrads umfasst, und wobei das Aggregat das Vorhandensein des Analyten indiziert.

2. Verfahren nach Anspruch 1, wobei die Mehrzahl von Mikropartikeln des zweiten Typs zur gleichen Zeit zur Probe hinzugefügt wird, wie die Mehrzahl von Mikropartikeln des ersten Typs zur Probe hinzugefügt wird.

3. Verfahren nach Anspruch 1, wobei die Mehrzahl von Mikropartikeln des zweiten Typs zur Probe hinzugefügt wird, nachdem die Mehrzahl von Mikropartikeln des ersten Typs zur Probe hinzugefügt wurde.

4. Verfahren nach Anspruch 1 oder 3, wobei die Mehrzahl von Mikropartikeln des zweiten Typs weniger als 10 Minuten, nachdem die Mehrzahl von Mikropartikeln des ersten Typs zur Probe hinzugefügt wurde, zur Probe hinzugefügt wird, wie z. B. wobei die Mehrzahl von Mikropartikeln des zweiten Typs weniger als 5 Minuten, nachdem die Mehrzahl von Mikropartikeln des ersten Typs zur Probe hinzugefügt wurde, zur Probe hinzugefügt wird.

5. Verfahren nach einem der Ansprüche 1 bis 4, wobei der erste Abschnitt des Analyten und der zweite Abschnitt des Analyten eine gemeinsame Region aufweisen.

6. Verfahren nach einem der Ansprüche 1 bis 4, wobei der erste Abschnitt des Analyten und der zweite Abschnitt des Analyten unabhängig sind.

7. Verfahren nach einem der Ansprüche 1 bis 6, wobei der Analyt eine Nukleinsäure, ein Polypeptid oder ein Polysaccharid ist.

8. Verfahren nach einem der Ansprüche 1 bis 7, wobei der erste Bindungspartner ein Kohlenhydrat, eine Nukleinsäure oder ein Polypeptid ist.

9. Verfahren nach einem der Ansprüche 1 bis 8, wobei der zweite Bindungspartner eine Nukleinsäure oder ein Polypeptid ist.

10. Verfahren nach einem der Ansprüche 1 bis 9, wobei der erste Bindungspartner durch eine kovalente, ionische, Van-der-Waals-, dipolare oder Wasserstoffbindung mit dem zumindest einen Mikropartikel des ersten Typs assoziiert ist, wie z. B. durch eine kovalente oder ionische Bindung.

11. Verfahren nach einem der Ansprüche 1 bis 10, wobei der zweite Bindungspartner durch eine kovalente, ionische, Van-der-Waals-, dipolare oder Wasserstoffbindung mit dem zumindest einen Mikropartikel des zweiten Typs assoziiert ist, wie z. B. durch eine kovalente oder ionische Bindung.

12. Verfahren nach einem der Ansprüche 1 bis 11, wobei das Identifizieren eines Aggregats umfasst:
Abbilden einer Kontrolle in Abwesenheit des Analyten, um ein Kontrollbild zu erzeugen;
Abbilden der Probe, die die Mehrzahl von Mikropartikeln des ersten Typs und die Mehrzahl von Mikropartikeln des zweiten Typs umfasst, um ein Probenbild zu erzeugen;
Zuweisen eines Farbskalawerts zu jedem Pixel des Kontrollbilds auf Basis der Intensität einer Farbe im Pixel;
Zuweisen eines Farbskalawerts zu jedem Pixel des Probenbilds auf Basis der Intensität einer Farbe im Pixel;
Berechnen einer Verteilung der Farbskalawerte, die den Pixeln auf dem Probenbild zugewiesen sind;
Berechnen einer Verteilung der Farbskalawerte, die den Pixeln auf dem Kontrollbild zugewiesen sind; und
Vergleichen der Verteilung der Farbskalawerte, die den Pixeln auf dem Probenbild zugewiesen sind, mit der Verteilung der Farbskalawerte, die den Pixeln auf dem Kontrollbild zugewiesen sind, wobei eine breitere Verteilung der Farbskalawerte, die den Pixeln auf dem Probenbild zugewiesen sind, als die Verteilung der Farbskalawerte, die den Pixeln auf dem Kontrollbild zugewiesen sind, das Vorhandensein eines Aggregats indiziert.

13. Verfahren nach Anspruch 12, das ferner das Strömen zumindest eines Tropfens einer Agglutinationsmischung durch einen Kanal umfasst, wie z. B. das ferner das Quantifizieren der Agglutination durch die Verteilung der Grauskalawerte des Bilds umfasst.

14. Verfahren nach Anspruch 13, wobei die Agglutinationsmischung durch funktionalisierte Mikroperlen und den Analyten gebildet wird, und vorzugsweise wobei die Durchflussrate zwischen 25 µl/min und 500 µl/min liegt.

15. Verfahren nach einem der Ansprüche 1 bis 11, das ferner das Messen eines Widerstands der Agglutinationsmischung umfasst.

16. Verfahren nach einem der Ansprüche 1 bis 11, wobei das Identifizieren eines Aggregats umfasst:
Abbilden der Probe, die die Mehrzahl von Mikropartikeln des ersten Typs und die Mehrzahl von Mikropartikeln des zweiten Typs umfasst, um ein Probenbild zu erzeugen;
Zuweisen eines binären Werts zu jedem Pixel des Probenbilds auf Basis der Intensität einer Farbe im Pixel; und
Berechnen der Anzahl von Pixeln auf dem Probenbild, die einem ersten binären Wert zugewiesen sind, wobei das Zuweisen des ersten binären Werts zu einem beliebigen Pixel das Vorhandensein eines Aggregats indiziert.

17. Verfahren nach Anspruch 16, wobei das Identifizieren eines Aggregats umfasst:
Abbilden einer Kontrolle in Abwesenheit des Analyten, um ein Kontrollbild zu erzeugen;
Zuweisen eines binären Werts zu jedem Pixel des Kontrollbilds auf Basis der Intensität einer Farbe im Pixel;
Berechnen der Anzahl von Pixeln auf dem Kontrollbild, die dem ersten binären Wert zugewiesen sind; und
Vergleichen der Anzahl von Pixeln auf dem Probenbild, die dem ersten binären Wert zugewiesen sind, mit der Anzahl von Pixeln auf dem Kontrollbild, die dem ersten binären Wert zugewiesen sind, wobei das Vorhandensein eines Aggregats indiziert ist, wenn die Anzahl von Pixeln auf dem Probenbild, die dem ersten binären Wert zugewiesen sind, größer als die Anzahl von Pixeln auf dem Kontrollbild ist, die dem ersten binären Wert zugewiesen sind.

18. Verfahren nach den Ansprüchen 1 bis 17, wobei das Quantifizieren des Aggregationsgrads das Berechnen des Prozentsatzes von Pixeln auf dem Bild der Probe umfasst, die dem ersten binären Wert zugewiesen sind.

## Revendications

1. Procédé de détection de la présence d'un analyte dans un échantillon, comprenant :
l'ajout d'une pluralité de microparticules d'un premier type à l'échantillon, où chaque microparticule du premier type comprend un premier partenaire de liaison configuré pour interagir avec au moins une première partie de l'analyte ;
l'ajout d'une pluralité de microparticules d'un second type à l'échantillon, où chaque microparticule du second type comprend un second partenaire de liaison configuré pour interagir avec au moins une seconde partie de l'analyte, la première partie de l'analyte étant différente de la seconde partie de l'analyte ; et
l'identification d'un agrégat comprenant au moins une microparticule du premier type, au moins une microparticule du second type et l'analyte, où l'identification d'un agrégat comprend la quantification du degré d'agrégation, et où l'agrégat indique la présence de l'analyte.

2. Procédé selon la revendication 1, dans lequel la pluralité de microparticules du second type est ajoutée à l'échantillon en même temps que l'ajout de la pluralité de microparticules du premier type à l'échantillon.

3. Procédé selon la revendication 1, dans lequel la pluralité de microparticules du second type est ajoutée à l'échantillon après l'ajout de la pluralité de microparticules du premier type à l'échantillon.

4. Procédé selon la revendication 1 ou 3, dans lequel la pluralité de microparticules du second type est ajoutée à l'échantillon moins de 10 minutes après l'ajout de la pluralité de microparticules du premier type à l'échantillon, tel que dans lequel la pluralité de microparticules du second type est ajoutée à l'échantillon moins de 5 minutes après l'ajout de la pluralité de microparticules du premier type à l'échantillon.

5. Procédé selon l'une quelconque des revendications 1 à 4, dans lequel la première partie de l'analyte et la seconde partie de l'analyte possèdent une région commune.

6. Procédé selon l'une quelconque des revendications 1 à 4, dans lequel la première partie de l'analyte et la seconde partie de l'analyte sont indépendantes.

7. Procédé selon l'une quelconque des revendications 1 à 6, dans lequel l'analyte est un acide nucléique, un polypeptide ou un polysaccharide.

8. Procédé selon l'une quelconque des revendications 1 à 7, dans lequel le premier partenaire de liaison est un glucide, un acide nucléique ou un polypeptide.

9. Procédé selon l'une quelconque des revendications 1 à 8, dans lequel le second partenaire de liaison est un acide nucléique ou un polypeptide.

10. Procédé selon l'une quelconque des revendications 1 à 9, dans lequel le premier partenaire de liaison est associé à la au moins une microparticule du premier type par une liaison covalente, ionique, de van der Waals, dipolaire ou hydrogène, tel que par une liaison covalente ou ionique.

11. Procédé selon l'une quelconque des revendications 1 à 10, dans lequel le second partenaire de liaison est associé à la au moins une microparticule du second type par une liaison covalente, ionique, de van der Waals, dipolaire ou hydrogène, tel que par une liaison covalente ou ionique.

12. Procédé selon l'une quelconque des revendications 1 à 11, dans lequel l'identification d'un agrégat comprend :
l'imagerie d'un contrôle en l'absence de l'analyte afin de créer une image de contrôle ;
l'imagerie de l'échantillon comprenant la pluralité de microparticules du premier type et la pluralité de microparticules du second type afin de créer une image de l'échantillon ;
l'attribution d'une valeur d'échelle de couleur à chaque pixel de l'image de contrôle basée sur l'intensité d'une couleur dans le pixel ;
l'attribution d'une valeur d'échelle de couleur à chaque pixel de l'image de l'échantillon basée sur l'intensité d'une couleur dans le pixel ;
le calcul d'une distribution des valeurs d'échelle de couleur attribuées aux pixels dans l'image de l'échantillon ;
le calcul d'une distribution des valeurs d'échelle de couleur attribuées aux pixels dans l'image de contrôle ; et
la comparaison de la distribution des valeurs d'échelle de couleur attribuées aux pixels dans l'image de l'échantillon avec la distribution des valeurs d'échelle de couleur attribuées aux pixels dans l'image de contrôle, où une plus grande distribution des valeurs d'échelle de couleur attribuées aux pixels dans l'image de l'échantillon que la distribution des valeurs d'échelle de couleur attribuées aux pixels dans l'image de contrôle indique la présence d'un agrégat.

13. Procédé selon la revendication 12, comprenant en outre la circulation d'au moins une gouttelette d'un mélange d'agglutination dans un canal, tel que comprenant en outre la quantification de l'agglutination par la distribution des valeurs d'échelle de gris de l'image.

14. Procédé selon la revendication 13, dans lequel le mélange d'agglutination est formé par des microbilles fonctionnalisées et l'analyte, et de préférence dans lequel le débit est compris entre 25 µl/min et 500 µl/min.

15. Procédé selon l'une quelconque des revendications 1 à 11, comprenant en outre la mesure de la résistance du mélange d'agglutination.

16. Procédé selon l'une quelconque des revendications 1 à 11, dans lequel l'identification d'un agrégat comprend :
l'imagerie de l'échantillon comprenant la pluralité de microparticules du premier type et la pluralité de microparticules du second type afin de créer une image de l'échantillon ;
l'attribution d'une valeur binaire à chaque pixel de l'image de l'échantillon basée sur l'intensité d'une couleur dans le pixel ; et
le calcul du nombre de pixels dans l'image de l'échantillon auxquels est attribuée une première valeur binaire, où l'attribution de la première valeur binaire à un quelconque pixel indique la présence d'un agrégat.

17. Procédé selon la revendication 16, dans lequel l'identification d'un agrégat comprend :
l'imagerie d'un contrôle en l'absence de l'analyte afin de créer une image de contrôle ;
l'attribution d'une valeur binaire à chaque pixel de l'image de contrôle basée sur l'intensité d'une couleur dans le pixel ;
le calcul du nombre de pixels dans l'image de contrôle auxquels est attribuée la première valeur binaire ; et
la comparaison du nombre de pixels dans l'image de l'échantillon auxquels est attribuée la première valeur binaire avec le nombre de pixels dans l'image de contrôle auxquels est attribuée la première valeur binaire, où la présence d'un agrégat est indiquée lorsque le nombre de pixels dans l'image de l'échantillon auxquels est attribuée la première valeur binaire est supérieur au nombre de pixels dans l'image de contrôle auxquels est attribuée la première valeur binaire.

18. Procédé selon les revendications 1 à 17, dans lequel la quantification du degré d'agrégation comprend le calcul du pourcentage de pixels dans l'image de l'échantillon auxquels est attribuée la première valeur binaire.
